# EUROPEAN PATENT SPECIFICATION

(11) **EP 2 794 812 B1**
(45) Date of publication and mention of the grant of the patent: **22.03.2017**
(21) Application number: 12813359.2
(22) Date of filing: 21.12.2012
(51) Int. Cl.: C09K 11/06, G01N 33/58, C07D 213/40

(54) **NOVEL LUMINESCENT LANTHANIDE CHELATES WITH ENHANCED EXCITATION PROPERTIES**
NEUARTIGE LUMINESZENTE LANTHANIDCHELATE MIT VERBESSERTEN ERREGUNGSEIGENSCHAFTEN
NOUVEAUX CHÉLATES LUMINESCENTS DE LANTHANIDE PRÉSENTANT DES PROPRIÉTÉS D'EXCITATIONS AUGMENTÉES

(30) Priority: 22.12.2011 DK 201100996; 22.12.2011 US 201161578977 P; 17.08.2012 WO PCT/EP2012/066082; 17.08.2012 US 201213589027
(43) Date of publication of application: 29.10.2014
(73) Proprietor: Radiometer Turku Oy, FI-20750 Turku (FI)
(72) Inventor: MELTOLA, Niko, FI-20760 Piispanristi (FI); SUND, Henri, FI-20500 Turku (FI); TAKALO, Harri, FI-20360 Turku (FI)
(74) Representative: Marnaes, Lene Meineche
(86) International application number: PCT/EP2012/076618
(87) International publication number: WO 2013/092992

(56) References cited:
- EP-A1- 0 967 205
- EP-A2- 1 447 666
- WO-A1-00/48990
- WO-A1-00/48991
- WO-A1-2006/026038
- WO-A1-2008/020113

## Description

### FIELD OF INVENTION

The invention relates to a novel lanthanide chelate design having substituted phenylethynyl pyridine chromophores around an emitting lanthanide ion. The substitution pattern in the phenylethynyl chromophores shifts the excitation spectrum to longer wavelengths and thereby enables efficient excitation of the lanthanide ion at wavelengths higher than 340 nm. The invention also relates to chelates to be attached to a biospecific reactant and their use in various assays.

### BACKGROUND OF INVENTION

Time-resolved fluorometry (TRF) employing long-lifetime emitting luminescent lanthanide chelates has been applied in many specific binding assays, such as e.g. immunoassays, DNA hybridization assays, receptor-binding assays, enzymatic assays, bio-imaging such as immunocytochemical, immunohistochemical assays or cell based assays to measure target analytes at very low concentration. Moreover, lanthanide chelates have been used in magnetic resonance imaging (MRI) and position emission tomography (PET).

For TRF application, an optimal label has to fulfill several requirements. First, it has to be photochemical stable both in the ground state and in the excited state and it has to be kinetically and chemically stable. The excitation wavelength has to be as high as possible, preferable over 300 nm. It has to have efficient cation emission i.e. high luminescence yield (excitation coefficient x quantum yield, εΦ). The observed luminescence decay time has to be long, and the chelate has to have good water solubility. For the purpose of labeling, it should have a reactive group to allow covalent attachment to a biospecific binding reactant, and the affinity and nonspecific binding properties of the labeled biomolecules have to be retained.

Although shifting of excitation wavelength has been successfully employed in scientific literature, the problem of existing technologies has been that the energy transfer from the chromophore to the lanthanide ion has been relatively weak. In other words shifting of excitation wavelength to longer wavelengths has typically been accompanied by weakening of the emission intensity. A majority of the published structures are also unsuitable to be used in aqueous environment i.e. not suitable to be used as labelling reagents in bioaffinity assays.

Knapton et al., "Fluorescent Organometallic Sensors for the Detection of Chemical-Warfare-Agent Mimics", Angew. Chem. Int. Ed. 2006, 45, 5825-5829, discloses a water-insoluble 2,4-dimethoxyphenylethynylenepyridine.

WO 2008/020113 A1 discloses luminescent lanthanide labelling reagents, e.g. 2,2',2",2"'-{[2-(4-isothiocyanatophenyl)ethylimino]bis(methylene)bis{4-[2-methoxy-4-(carboxymethoxy)phenyl]pyridine-6,2-diyl}bis(methylenenitrilo)}-tetrakis(acetato)} terbium(III) and their use.

EP 1 447 666 A2 relates to a luminescent lanthanide chelate comprising a lanthanide ion and a chelating ligand of formula (I) wherein R1 is selected from the group consisting H, -COOH, -COO-, -CH2COOH and -CH2COO-, G1 is a group consisting of one or two moieties each moiety being selected from the group consisting of ethynediyl, ethenylene, phenylene, biphenylene, naphthylene, pyridylene, pyrazinylene,pyrimidinylene, pyridazinylene, furylene, thienylene, pyrrolylene, imidazolylene, pyrazolylene, thiazolylene, isothiazolylene, oxazolylene, isoxazolylene, fyrazanylene, 1,2,4-triazol-3,5-ylene and oxadiazolylene; G2 for coupling to a biospecific binding reactant is selected from the group consisting of amino, aminooxy, carbonyl, aldehyde or mercapto groups and activated forms made of them; Z is selected from the group consisting of carboxyalkyl amine, ether, thioether, carbonyl and unsubstituted or substitute methyl (-CR2-) wherein group R2 is selected from the group consisting of H, methyl, ethyl and carboxylalkyl; and the lanthanide ion is europium(III), terbium(III), dysprosium(III) or samarium(III). EP 1 447 666 A2 further relates to a detectable molecule comprising the lanthanide chelate and the use of the molecule in a method of carrying out a biospecific binding assay.

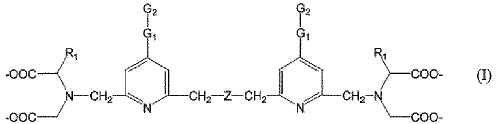

The structures according to the present invention provide a general method for shifting the excitation wavelength to above 340 nm without compromising the chelate performance, i.a. with respect to luminescence yield. The present invention thereby enables the use of cheap UV LEDs as an excitation source. Currently 365 nm UV LEDs with sufficient excitation energy are available and the excitation spectrum of the chelates according to the present invention strongly suggests the applicability of these chelates in combination with such UV LEDs. Application of LEDs in the instrument design enables cost reduction and miniaturization of the instrument.

Also, the structures according to the invention offers highly luminescent water soluble chelate structures with high excitation wavelength although the chromophore(s) has substituted alkoxy substituents in both mesomeric orto- and para- positions. The adsorption properties of the chromophore moieties are decreased due to the additional hydrophilic groups near to the long aromatic π-electron containing structure. This means that these labels should not suffer from increased background although even three independent chromophores are present in the ligand structure.

### SUMMARY OF INVENTION

A first aspect of the invention relates to a luminescent lanthanide chelate comprising one or more chromophoric moieties of the formula (I) or of the formula (III)

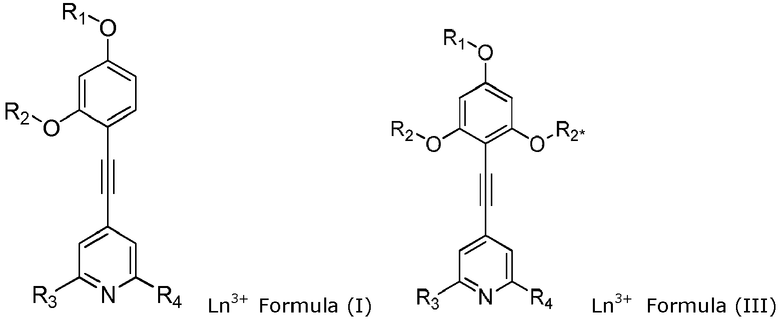

A second aspect of the invention relates to a detectable molecule comprising a biospecific binding reactant conjugated to a luminescent lanthanide chelate comprising one or more moieties of the formula (I) as defined herein.

A third aspect of the invention relates to a luminescent lanthanide chelating ligand comprising one or more chromophoric moieties of the formula (II) or of the formula (IV)

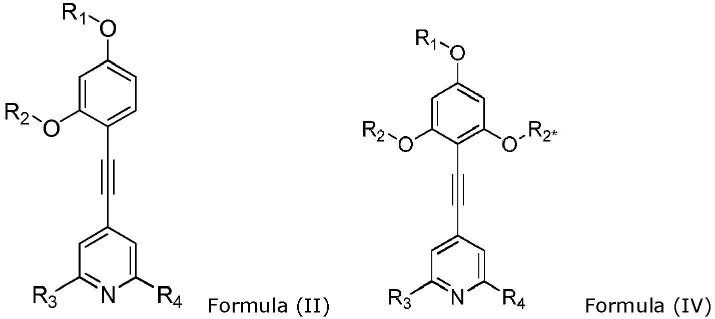

A fourth aspect of the invention relates to a method of carrying out a biospecific binding assay, said method comprising the steps of: a) forming a biocomplex between an analyte and a biospecific binding reactant labelled with a luminescent lanthanide chelate as defined herein; b) exciting said biocomplex with radiation having an excitation wavelength, thereby forming an excited biocomplex; and c) detecting emission radiation emitted from said excited biocomplex.

A fifth aspect of the invention relates to the use of a detectable molecule as defined herein in a specific bioaffinity based binding assay utilizing time-resolved fluorometric determination of a specific luminescence.

A sixth aspect of the invention relates to a solid support material conjugated with a luminescent lanthanide chelate as defined herein.

The structural modification according to the present invention has been employed in several different chelate structures (**14, 20** and **30**; see the Examples). For these chelates where the modification was introduced, a more than 20 nm shift of the excitation spectrum was observed compared to chelates having a conventional substitution pattern.

### DETAILED DESCRIPTION OF INVENTION

The aim of the present invention is to provide means to obtain improved lanthanide chelate labels to be used in specific bioaffinity based binding assays, such as immunoassays (both homogeneous and heterogeneous), nucleic acid hybridization assays, receptor-binding assays, enzymatic assays, immunocytochemical, immunohistochemical assays and cell based assays utilizing fluorometric or time-resolved fluorometric determination of specific luminescence. Chelates of the present invention provide means to obtain improved bioaffinity based binding assays related to e.g. assay sensitivity, kinetics and background, even at wavelengths above 340 nm.

### Luminescent lanthanide chelate

One aspect of the present invention relates to a luminescent lanthanide chelate comprising one or more chromophoric moieties of the formula (I) or of the formula (III) 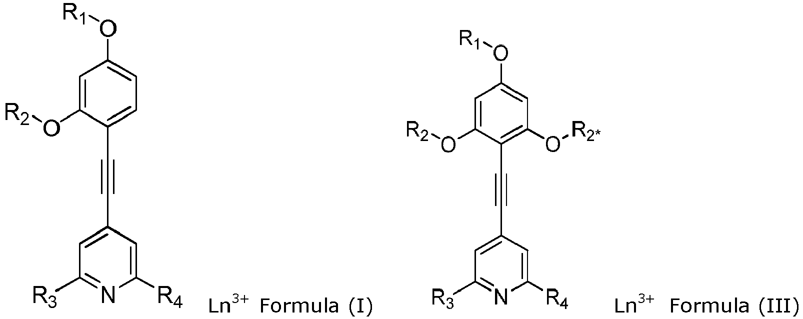 wherein R₁, R₂ and R₂*, when present, each independently are selected from carbon-containing substituents forming a C-O bond with the neighbouring oxygen atom, R₃ and R₄ each represent a bond between the chromophoric moiety and other moieties of the chelate, and Ln³⁺ is a lanthanide ion.

It has been found that the 2,4-(R-O)-substituent configuration (i.e. formula (I)) as well as the 2,4,6-(R-O)-substituent configuration (i.e. formula (III)) provide a significant shift in the excitation spectrum to longer wavelengths compared to a reference chelate of an otherwise similar type, but with nor or little reduction in the emission intensity.

The substituents R₁, R₂ and R₂* each independently are selected from certain carbon-containing substituents forming a C-O bond with the neighbouring oxygen atom. It is believed that the specific substituent configuration combined with the substituent type (R-O) is responsible for the observed effect. On the other hand, the substituents R₁, R₂ and R₂* are believed to render the lanthanide chelate comparably more water-soluble and are believed to reduce the unspecific binding properties and thereby reduce the background signal.

Hence, R₁, R₂ and R₂*, when present, are each independently selected
from -(CH₂)₁₋₆COOH, -(CH₂)₁₋₆COO⁻, -(CH₂)₁₋₆SO₃H, -(CH₂)₁₋₆SO₃⁻, -(CH₂CH₂O)₁₋₄OCH₂CH₂OH, -(CH₂CH₂O)₁₋₄OCH₂CH₂OCH₃, -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, -(CH₂)₁₋₆C(=O)R₅, and -(CH₂)₁₋₆-C₆H₄-R₅, wherein R₅ is selected from hydrogen, C₁₋₁₂-alkyl (in particular C₁₋₆-alkyl), -(CH₂)₁₋₆COOH, -(CH₂)₁₋₆COO-, -(CH₂)₁₋₆SO₃H, -(CH₂)₁₋₆SO₃⁻, a hydrophilic group (optionally including a spacer), a reactive group (optionally including a spacer), a polypeptide, and a polynucleotide. In one embodiment, R₁, R₂ and R₂*, when present, are each independently selected from -(CH₂)₁₋₆COOH, -(CH₂)₁₋₆COO⁻, -(CH₂)₁₋₆SO₃H, -(CH₂)₁₋₆SO₃⁻, -(CH₂CH₂O)₁₋₄OCH₂CH₂OH and -(CH₂CH₂O)₁₋₄OCH₂CH₂OCH₃, -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, -(CH₂)₁₋₆C(=O)R₅, wherein R₅ is selected from hydrogen, C₁₋₁₂-alkyl (in particular C₁₋₆-alkyl), -(CH₂)₁₋₆COOH, -(CH₂)₁₋₆COO-, -(CH₂)₁₋₆SO₃H, and -(CH₂)₁₋₆SO₃⁻.

In another embodiment, one of R₁, R₂ and R_{2*}, when present, is -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, -(CH₂)₁₋₆C(=O)R₅, and -(CH₂)₁₋₆-C₆H₄-R₅, wherein R₅ is a reactive group (optionally including a spacer), in particular R₅ is -NCS, while the other of R₁, R₂ and R_{2*}, when present, are as defined above, except that any R₅ is not a reactive group. In one variant hereof, one of R₁, R₂ and R_{2*}, when present, is -(CH₂)₁₋₆-C₆H₄-NCS, in particular -CH₂-C₆H₄-NCS.

It is presently believed that some types of substituents, i.e. those of the carboxylic acid and sulfonic acid type, are especially interesting. Hence, in a preferred embodiment, R₁, R₂ and R_{2*} (when present) are each independently selected from -(CH₂)₁₋₆COOH, -(CH₂)₁₋₆COO⁻,-(CH₂)₁₋₆SO₃H, and -(CH₂)₁₋₆SO₃⁻, in particular from -CH₂-COOH and -CH₂-COO⁻.

In one embodiment R₁, R₂ and R₂* (when present) are each independently selected from-CH₂-COOH and -CH₂-COO⁻.

In one embodiment, the one or more chromophoric moieties are of the formula (I), i.e. having a 2,4-substitution pattern. It is believed that such a substitution pattern provides a reduction of the unspecific binding properties (reduction of the background signal).

In another embodiment, the one or more chromophoric moieties of the formula (III), i.e. having a 2,4,6-substitution pattern. It is believed that such a substitution pattern provides a reduction of the unspecific binding properties (reduction of the background signal.

It should be understood that when the substituents are carboxylates, sulfonates and the like, the chelates may include cations as counter ions, e.g. Na⁺, K⁺, Ca²⁺ and the like.

When present, any hydrophilic groups are present in order to, e.g., improve water solubility of the chelate.

Examples of hydrophilic groups are mono- and oligosaccharides, such as monosaccharides and disaccharides, oligoalkylene glycols (e.g. those having 1-20 repeating units) such as oligoethylene glycol and oligopropylene glycol, etc.

In one embodiment, the hydrophilic group is selected from monosaccharides, disaccharides, -C(CH₂OH)₃-(CH₂)₁₋₃-O-(CH₂CH₂O)₀₋₅-H, -(CH₂)₁₋₃-O-(CH₂CH₂O)₀₋₅-C₁₋₄-alkyl, -O-(CH₂CH₂O)₁₋₆-H, and -O-(CH₂CH₂O)₁₋₆-C₁₋₄-alkyl, in particular monosaccharides.

In the present context, the term "monosaccharide" is intended to mean C₅-C₇ carbohydrates being either in the acyclic or in cyclic form. Examples of monosaccharides are C₆ carbohydrates, e.g. those selected from 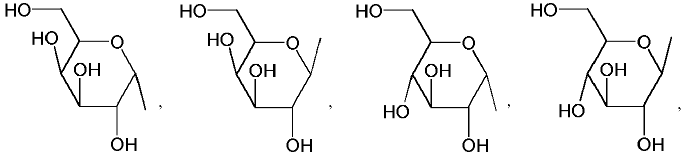 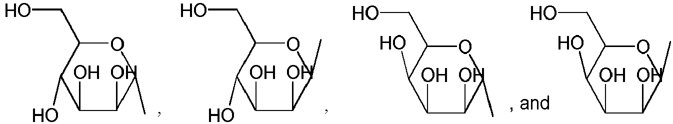 or

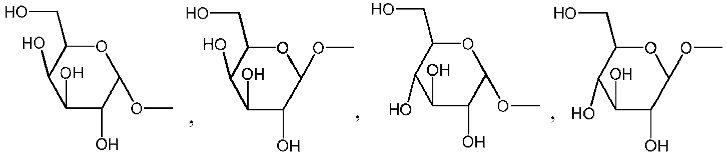

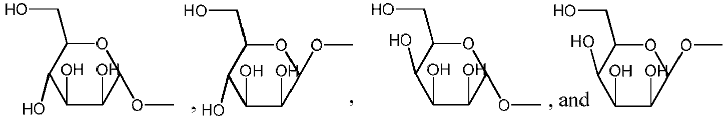

In the present context, the term "disaccharide" is intended to mean two monosaccharides (cf. above) linked together, preferably via glycosidic bonds.

In other possible embodiments, a hydrophilic group (as specified) is present in the chelate structure, but not in the chromophoric moiety of formula (I).

In some alternative embodiments, the substituent R₁ and/or R₂ and/or R_{2*} includes a reactive group Z, preferably including a spacer (see further below). In such instances, the reactive group Z is facilitating the labelling of a biospecific binding reactant, or is facilitating the formation of a covalent bond to a solid support material. In case the chelate has a polymerizing group as reactive group, then the chelate may be introduced in the solid support, e.g. a particle, simultaneously with the preparation of the particles.

If present, the reactive group Z is typically selected from azido (-N₃), alkynyl (-C≡CH), alkylene (-CH=CH₂), amino (-NH₂), aminooxy (-O-NH₂), carboxyl (-COOH), aldehyde (-CHO), mercapto (-SH), maleimido, activated derivatives of maleimido, isocyanato (-NCO), isothiocyanato (-NCS), diazonium (-N⁺N), bromoacetamido, iodoacetamido, reactive esters, pyridyl-2-dithio, and 6-substituted 4-chloro-1,3,5-triazin-2-ylamino, in particular, the reactive group comprises a isothiocyanato (-NCS) group. The substituents in 6-substituted 4-chloro-1,3,5-triazin-2-ylamino can be selected from the group consisting hydrogen, halogen, alkoxy, aryloxy, amino, alkyl with one to six carbon atoms, substituted amino or thioethers, and preferable selected from the group consisting of chloro, fluoro, ethoxy, 2-methoxyethoxy, 2-cyanoethoxy, 2,2,2-trifluoroethoxy, thiophenoxy or ethoxycarbonyl-thiomethoxy. The substituted amino or thioether is preferable mono- or disubstituted each substituent being preferable independently selected from the group consisting of an alkyl or alkoxy with one to six carbon atoms, phenyl, carbonyl or carboxyl.

It follows that upon reaction with a biospecific binding reactant (see further below), the reactive group Z establishes a link to said biospecific binding reactant, e.g. of one of the following types: a thiourea (-NH-C(=S)-NH-), an aminoacetamide (-NH-CO-CH₂-NH-), an amide (-NH-CO-, -CO-NH-, -NCH₃-CO- and -CO-NCH₃-), and aliphatic thioether (-S-), a disulfide (-S-S-), a 6-substituted-1,3,5-triazine-2,4-diamine, a 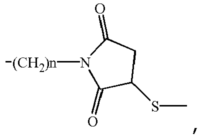 wherein n = 1-6; and a triazole (e.g. formed by the so-called "click" chemistry).

In other possible embodiments, a reactive group Z (as specified) is present in the chelate structure, but not in the chromophoric moiety of formula (I).

It should be understood that when a reactive group Z is present, the group Z may include a spacer, i.e. a distance-making biradical, so as - if necessary or desirable - to position the reactive group Z in a position accessible for reaction with the biospecific binding reactant. Similarly, when any of R₁, R₂ and R_{2*} include a hydrophilic group, the hydrophilic group may include a spacer. In both instances, the spacer may be readily introduced in the course of the synthesis of the ligand or the chelate.

The term "spacer" is intended to mean a distance-making group between, e.g., a conjugating group or a pyridine moiety of the core structure and, e.g. the reactive group Z or a hydrophilic group. The spacer typically has a length of 1-20 bonds between the attachment point and reactive group (or hydrophilic group), such as 3-15 bonds, or 5-12 bonds. The said spacer is formed of one to five moieties, each moiety selected from the group consisting of phenylene, alkylene containing 1-10 carbon atoms, an ethynediyl (-C≡C-), an ether (-O-), a thioether (-S-), a disulfide (-S-S-), an amide (-C(=O)-NH-, -NH-C(=O)-, -C(=O)-NCH₃- and - NCH₃-C(=O)-), a thiourea (-NH-C(=S)-NH-) and a triazole.

In some embodiments, at least one of the substituents R₁ and/or R₂ and/or R_{2*} include apolypeptide or a polynucleotide.

R₃ and R₄ each represent a bond between the chromophoric moiety and other moieties of the chelate, e.g. chromophoric moieties and chelating moieties. The chelating moity comprising at least two carboxylic acid or phosphoric acid groups, ester, amides or salts of said acids, attached to an aromatic unit of the chromophoric moiety, either directly or via a cyclic or acyclic N- and/or O-containing hydrocarbon chain. It should be understood that the chromophoric moiety of formula (I) or (II) may replace any phenylethynylpyridine moiety in conventional chelates. Hence, it is appreciated that the chromophoric moiety can simply be incorporated in conventional chelates having other chromophoric moieties either similar or different to the chromophoric moiety of formula (I) or (II). Illustrative examples are provided in the examples section.

R₃ and R₄ each represent a bond to other moieties of the chelate, e.g. to another chromophoric moiety, typically via a linker, or to a complexing group (e.g. -COOH / -COO⁻), or simply to an end group or a hydrogen atom.

In some interesting embodiments, the chelate has a total of two or three chromophoric groups, e.g. as illustrated with the Formulas (A) and (B).

The term "lanthanide ion" or "Ln³⁺" is intended to mean a trivalent ion of the lanthanide series of the Periodic Table of Elements, e.g. europium(III), terbium(III), samarium(III) and dysprosium(III), i.e. Eu³⁺, Tb³⁺, Sm³⁺ or Dy³⁺. In many embodiments europium(III) (Eu³⁺) is preferred.

Moreover, the invention provides highly luminescent labels for all lanthanides which provides multi-label possibilities.

### Particular embodiments

In some particular embodiments, the chelate has one of the structural formulae (A-I), (A-II), (B-I) and (B-II) below:

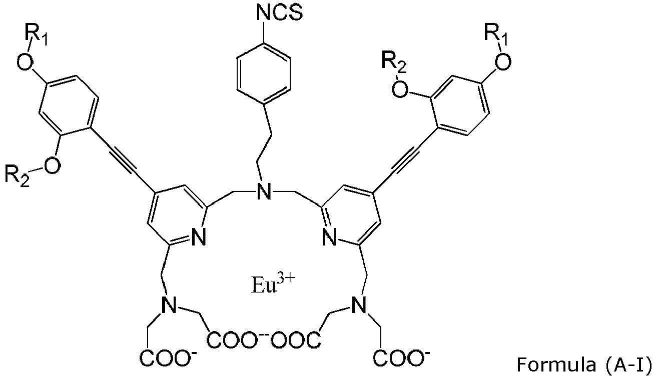

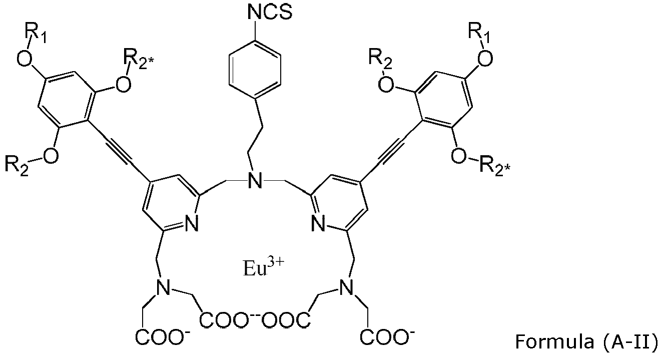

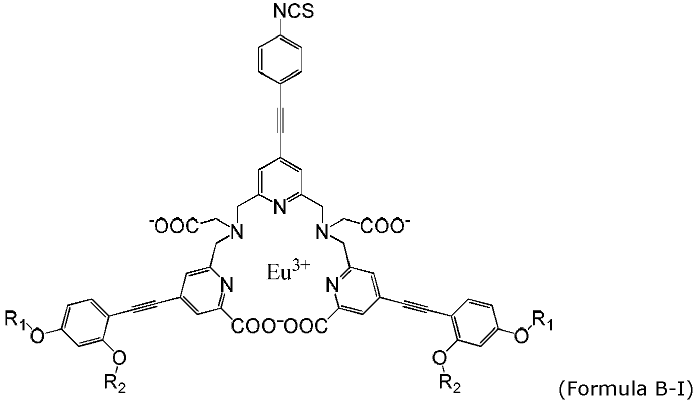

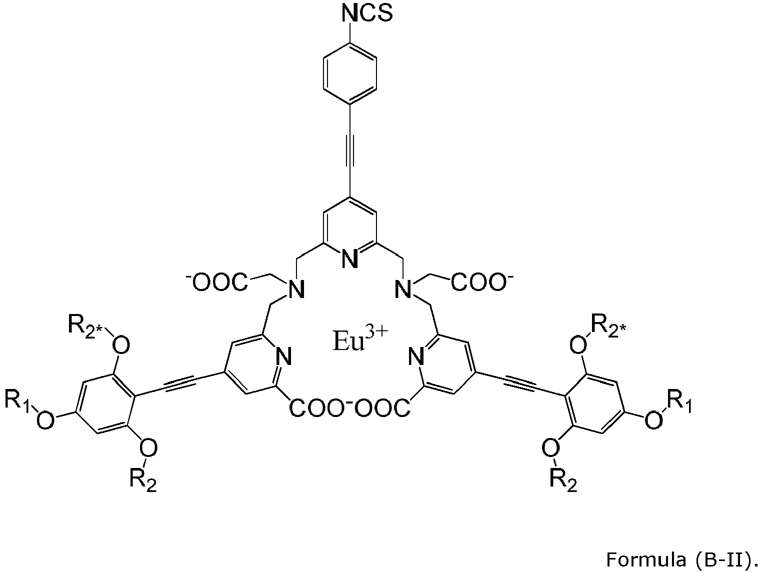

In each of the formulas (A-I), (A-II), (B-I) and (B-II), the substituents R₁, R₂ and R_{2*}, when present, may each independently be selected from -(CH₂)₁₋₆COOH, -(CH₂)₁₋₆COO⁻, -(CH₂)₁₋₆SO₃H, -(CH₂)₁₋₆SO₃⁻, -(CH₂CH₂O)₁₋₄OCH₂CH₂OH, -(CH₂CH₂O)₁₋₄OCH₂CH₂OCH₃, -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, and -(CH₂)₁₋₆C(=O)R₅, wherein R₅ is selected from hydrogen, C₁₋₁₂-alkyl, -(CH₂)₁₋₆COOH, -(CH₂)₁₋₆COO⁻, -(CH₂)₁₋₆SO₃H, -(CH₂)₁₋₆SO₃⁻, a hydrophilic group (optionally including a spacer), a reactive group (optionally including a spacer), a polypeptide and a nucleotide.

In one preferred embodiment, the chelate has the formula (A-I) or (B-I) and R₁ and R₂ are each independently selected from -(CH₂)₁₋₆COOH, -(CH₂)₁₋₆COO⁻, -(CH₂)₁₋₆SO₃H, and -(CH₂)₁₋₆SO₃-, in particular from -CH₂-COOH and -CH₂-COO⁻.

In another preferred embodiment, the chelate has the formula (A-II) or (B-II) and R₁, R₂ and R_{2*} are each independently selected from -(CH₂)₁₋₆COOH, -(CH₂)₁₋₆COO⁻, -(CH₂)₁₋₆SO₃H, and -(CH₂)₁₋₆SO₃⁻, in particular from -CH₂-COOH and -CH₂-COO⁻.

It is further envisaged, that although Eu³⁺ is preferred in formula (A) and (B), it may be replaced by any other lanthanide selected from Tb³⁺, Sm³⁺ or Dy³⁺.

Particularly interesting are the lanthanide chelates of any one of the formulas **13, 14, 43, 19, 20, 44, 29, 30,** and **31.**

### Lanthanide chelating ligand

Hence, another aspect of the present invention relates to a lanthanide chelating ligand comprising one or more chromophoric moieties of the formula (II) or of the formula (IV) 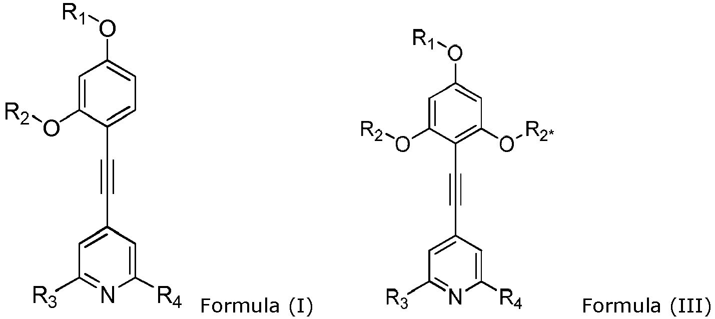 wherein each of R₁, R₂, R_{2*}, R₃, and R₄ represents the groups R₁, R₂, R_{2*}, R₃, and R₄, respectively, as defined for formula (I).

In some interesting embodiments, lanthanide chelating ligand has one of the formulas (A-I), (A-II), (B-I) or (B-II) above (excluding the Eu³⁺). Particularly interesting are the lanthanide chelates ligands of any one of the formulas **13, 14, 43, 19, 20, 44, 29, 30,** and **31** (excluding the Eu³⁺).

### A detectable molecule

Still another aspect of the present invention relates to a detectable molecule comprising a biospecific binding reactant conjugated to a luminescent lanthanide chelate as defined hereinabove. Conjugation is typically obtained by means of a reactive group of said chelate.

The biospecific binding reactant should be capable of specifically binding an analyte of interest for the purpose of quantitative or qualitative analysis of said analyte in a sample.

Examples of biospecific binding reactants are those selected from an antibody, an antigen, a receptor ligand, a specific binding protein, a DNA probe, a RNA probe, an oligopeptide, an oligonucleotide, a modified oligonucleotide (e.g. an LNA modified oligonucleotide), a modified polynucleotide (e.g. an LNA modified polynucleotide), a protein, an oligosaccaride, a polysaccharide, a phospholipid, a PNA, a steroid, a hapten, a drug, a receptor binding ligand, and lectine.

In a preferred embodiment, the biospecific binding reactant is selected from antibodies, e.g. Troponin I antibodies (anti-TnI).

### A method for carrying out a biospecific binding assay

A still further aspect of the invention relates to a method of carrying out a biospecific binding assay, wherein the method comprises the steps of:
a) forming a biocomplex between an analyte and a biospecific binding reactant labelled with a lanthanide chelate as defined herein;
b) exciting said biocomplex with radiation having an excitation wavelength, thereby forming an excited biocomplex; and
c) detecting emission radiation emitted from said excited biocomplex.

In step b), the excitation wavelength is preferably 300 nm or longer, e.g. around 320-360 nm.

The method follows the conventional assay steps as will be evident for the skilled person.

This being said, a further aspect of the invention relates to the use of a detectable molecule as defined above in a specific bioaffinity based binding assay utilizing time-resolved fluorometric determination of a specific luminescence. In one embodiment, the specific bioaffinity based binding assay is a heterogeneous immunoassay, a homogenous immunoassay, a DNA hybridization assay, a receptor binding assay, an immunocytochemical or an immunohistochemical assay.

### A solid support

Still another aspect of the invention relates to a solid support material conjugated with a luminescent lanthanide chelate as defined hereinabove. The luminescent lanthanide chelate is typically immobilized to the solid support material either covalently or non-covalently.

In some interesting embodiments, the solid support material is selected from a nanoparticle, a microparticle, a slide, a plate, and a solid phase synthesis resin.

The novel lanthanide chelates ligands and the corresponding luminescent lanthanide chelates and labeled biospecific binding reactant are based on an open chain, i.e. acyclic, ligand structure which provides surprisingly efficiently excitation of the chelated lanthanide ion. At the same time, all important features of the luminescent lanthanide chelate and labeled biospecific binding reactant can be retained without any additional formation of aggregates and purification problems.

The chelates of the present invention aim to combine several important features in a single label such as:
(a) The shift towards longer wavelengths (see the Examples) enables the use of UV LEDs as an excitation source which will provide a cost reduction in instrument manufacturing, and the possibility of instrument miniaturization.
(b) The chelates are applicable to different lanthanides.
(c) It is possible to decrease the labeling degree without loss of signal.
(d) The lower degree of labeling can improve the affinity of the biomolecule and decrease unspecific binding during the assay. Thus faster kinetic is possible and lower background is seen which can also improve the assay sensitivity.
(e) Reduction of unwanted adsorption properties of the chromophore moiety with improved aqueous solubility, especially concerning chelates with several aromatic chromophore moieties. This should reduce the unspecific binding of the labeled antibody and give improved assay sensitivity.

### EXAMPLES

The following non-limiting examples are aimed to further demonstrate the invention.

¹H-NMR spectra were recorded with Bruker AVANCE DRX 500 MHz. Tetramethyl silane was used as internal reference. Mass spectra were recorded on Applied Biosystems QSTAR XL ESI-TOF instrument. UV-Vis spectra were recorded on Pharmacia Ultrospec 3300 pro. Fluorescence efficiencies were determined with Perkin-Elmer Wallac Victor™ plate-fluorometer. Column chromatography was performed with columns packed with silica gel 60 (Merck).

### Preparation of compounds

### Example 1. Synthesis of diethyl 2,2'-((4-bromo-1,3-phenylene)bis(oxy))diacetate (2)

4-bromoresorcinol (1.00 g, 5.29 mmol) was dissolved in DMF (20 ml, dry). Anhydrous K₂CO₃ (4.39 g, 31.74 mmol) and ethyl bromoacetate (2.30 ml, 15.87 mmol) were added. Mixture was stirred overnight at 40°C under argon atmosphere. Water (30 ml) was added and the mixture was extracted with ethyl acetate (1x 20 ml, 2x10 ml). Combined organic extracts were washed with water (2x10 ml), dried over Na₂SO₄ and concentrated. Crude product was purified by column chromatography using silica gel as stationary phase and dichloromethane as eluent. Product was a white solid. Yield: 1.70 g (89%) ¹H NMR (CDCl₃, δ ppm): 7.43 (1H, d, J=8.7Hz), 6.48 (1H, d, J=2.7Hz), 6.40 (1H, dd, J=8.7Hz, J=2.7Hz), 4.66 (2H, s), 4.57 (2H, s), 4.27 (4H, m) 1.30 (6H, m).

### Example 2. Synthesis of diethyl 2,2'-((4-((trimethylsilyl)ethynyl)-1,3-phenylene)bis(oxy))diacetate (3)

Compound **2** (1.56 g, 4.33 mmol) was dissolved in DMF (3 ml, dry) and the solution was placed in a microwave reaction vial. Diethyl amine (9 ml, dry), Pd(PPh₃)₂Cl₂ (152.0 mg, 0.217 mmol), CuI (41.2 mg, 0.217 mmol) and PPh₃ (113.6 mg, 0.433 mmol) were added and the vial was sealed in an argon atmosphere. Trimethylsilyl acetylene (925 µl, 6.50 mmol) was added through a septum and the mixture was stirred at 100°C for 30 minutes using microwave heating. Reaction mixture was filtrated through silica gel using dichloromethane as eluent and the filtrate was evaporated to dryness. The crude product was dissolved in dichloromethane and purified by column chromatography using silica gel as stationary phase and 10% ethyl acetate:petroleum ether as eluent. Yield: 0.99 g (61%). ¹H NMR (CDCl₃, δ ppm): 7.36 (1H, dd, J=1.35Hz, J=7.55Hz), 6.43 (2H, m), 4.67 (2H, s), 4.58 (2H, s), 4.27 (4H, m), 1.30 (6H, m), 0.25 (9H, s).

### Example 3. Synthesis of diethyl 2,2'-((4-ethynyl-1,3-phenylene)bis(oxy))diacetate (4)

Compound **3** (398.9 mg, 1.054 mmol) was dissolved in dichloromethane (10 ml, dry). Tetrabutyl ammoniumfluoride (330.7 mg, 1.265 mmol) was added and the mixture was stirred in argon atmosphere at room temperature for 1 h 30 min. Mixture was washed with 10% citric acid solution (5 ml), and water (4x10 ml). The organic phase was dried over Na₂SO₄ and evaporated to dryness. Crude product was purified by column chromatography using silica gel as stationary phase and 15% ethyl acetate:petroleum ether as eluent. Yield 259.2 mg (80%). ¹H NMR (CDCl₃, δ ppm): 7.40 (1H, d, J=8.35Hz), 6.45 (1H, d, J=2.4Hz), 6.43 (1H, dd, J=5.25Hz, J=2.4Hz), 4.70 (2H, s), 4.59 (2H, s), 4.27 (4H, m) 3.24 (1H, s), 1.29 (6H, m).

### Example 4. Synthesis of 2,6-bis(hydroxymethyl)-4-iodopyridine (6)

Diethyl 4-iodo-2,6-pyridinedicarboxylate (**5**) (5.57 g, 17.3 mmol) was suspended in ethanol (130 ml). Sodium borohydride (2.95 g, 78 mmol) was added in small portions to the stirred solution during 15 min. The mixture was refluxed for 1h 30 min and allowed then to cool down to RT. The reaction mixture was evaporated to dryness and the residue was suspended in saturated aqueous sodium hydrogen carbonate (35 ml). The suspension was rapidly boiled up, allowed to cool and evaporated to dryness. The residue was suspended in mixture of DMF and dichloromethane (1:1, 65 ml), filtrated through Celite pad and the filtrate was evaporated to dryness. The product was crystallized from water and dried in vacuum desiccator over silica gel. Yield 3.37 g (60%). 1HNMR (CDCl3, δ ppm): 7.70 (2H, s), 5.46 (2H, s), 4.48 (4H, s).

Compound **5** was prepared as described in Takalo H. and Kankare J., Acta Chemica Scandinavica B 41, 1987, 219-221.

### Example 5. Synthesis of 2,6-bis(bromomethyl)-4-iodopyridine(7)

Phosphorus tribromide (2.45 ml, 26 mmol) was added drop-wise to anhydrous DMF (19.5 ml) at 0 °C resulting in a semisolid mixture. 2,6-bis(hydroxymethyl)-4-iodopyridine (**6**) (3.36 g, 12.7 mmol) was added to the mixture in small portions. The solid mixture solubilizes during the addition. The mixture was allowed to warm up to RT and stirred 4 h at RT. The reaction mixture was poured to 100 ml of aqueous sodium hydrogen carbonate (5%). The precipitated product was collected by filtration, washed with water and dried in vacuum desiccator over silica gel. Yield 4.51g (91%). 1HNMR (CDCl3, δ ppm): 7.76 (2H, s), 4.45 (4H, s)

### Example 6. Synthesis of compound ethyl-2-((4-bromo-6-(carboxyethyl)-pyridine-2-yl)methylenenitrilo)acetate (9)

4-bromo-6-bromomethyl-2-carboxyethylpyridine (**8**) (2.99 g, 9.3 mmol) and glycine ethyl ester hydrochloride (6.52 g, 46.7 mmol) were dissolved in mixture of anhydrous acetonitrile (125 ml) and di-isopropylethylamine (16.5 ml). Mixture was stirred overnight at RT. Reaction mixture was evaporated to dryness and the product was purified with column chromatography using silica as a stationary phase and dichloromethane:methanol (3%97:3) as an eluent. Yield 2.78 g (86%). 1HNMR: (CDCl3, δ ppm): 8.14 (1H, d, J=1.65Hz), 7.85 (1H, d, J=1.60Hz), 4.47 (2H, q, J=7.12Hz), 4.20 (2H, q, J=7.13Hz), 4.05 (2H, s), 3.47 (2H, s), 2.26 (1H, s), 1.43 (3H, t, J=7.13Hz), 1.28 (3H, t, J=7.13Hz).

Compound **8** was prepared as described in Takalo et al., Helv. Chim. Acta, 1996, 79, 789-802.

### Example 7. Synthesis of diethyl 6,6'-((((4-iodopyridine-2,6-diyl)bis(methylene))bis((2-ethoxy-2-oxoethyl)azanediyl))bis(methylene))bis(4-bromopicolinate) (10)

Compound **7** (0.94 g, 2.4 mmol) and compound **9** (1.66 g, 4.8 mmol) were dissolved in anhydrous acetonitrile (80 ml). Anhydrous potassium carbonate (1.67 g, 12 mmol) was added and the mixture was stirred 4 hours at 55-60 °C. Reaction mixture was allowed to cool down to RT and filtrated through Celite pad. The filtrate was evaporated to dryness and the product was purified with column chromatography using silica as a stationary phase and dichloromethane:methanol (5%95:5) as an eluent. Yield 1.57 g (71%). 1HNMR: (CDCl3, δ ppm): 8.12 (2H, d, J=1.60Hz), 8.07 (2H, d, J=1.40Hz), 7.76 (2H, s), 4.46 (4H, q, J=7.12Hz), 4.18 (4H, q, J=7.13), 4.08 (4H, s), 3.92 (4H, s), 3.49 (4H, s), 1.42 (6H, t, J=7.13), 1.28 (6H, t, J=7.13). MS(ESI-TOF) calculated for C33H38Br2IN5O8 [M+H]+: 918.02, found: 917.92

### Example 8. Synthesis of diethyl 6,6'-((((4-((4-aminophenyl)ethynyl)pyridine-2,6-diyl)bis(methylene))bis((2-ethoxy-2-oxoethyl)azanediyl))bis(methylene))bis(4-bromopicolinate) (11)

Compound **10** (412 mg, 0.45mmol) was dissolved in anhydrous dichloromethane (6 ml). Triethylamine (2 ml), copper (I) iodide (3.2 mg, 0.017 mmol), bis(triphenylphosphine)palladium(II) dichloride (9.5 mg, 0.014 mmol) and 4-ethynylaniline (52.5 mg, 0.45 mmol) were added. The reaction mixture was de-aerated with argon and the mixture was stirred 3 h at RT. Reaction mixture was evaporated to dryness and the product was purified with column chromatography using silica as a stationary phase and dichloromethane:methanol (10% 90:10) as an eluent. Yield 193 mg (47%). 1HNMR: (CDCl3, δ ppm): 8.11 (2H, d, J=1.35Hz), 7.83 (2H, s), 7.32 (2H, d, J=8.15Hz), 7.18 (2H, s), 6.67 (2H, d, J=8.45Hz) 4.38 (4H, s), 4.30 (4H, q, J=6.74Hz), 4.01 (4H, q, J=7.10Hz), 3.97 (4H, s), 3.39 (4H, s), 1.32 (6H, t, J=6.90Hz), 1.17 (6H, t, J=6.93Hz). MS(ESI-TOF) calculated for C41H44Br2N6O8 [M+H]+: 907.17, found: 907.05

### Example 9. Synthesis of tetraethyl 2,2',2",2"'-((((6,6'-((((4-((4-aminophenyl)ethynyl)pyridine-2,6-diyl)bis(methylene))bis((2-ethoxy-2-oxoethyl)azanediyl))bis(methylene))bis(2-(ethoxycarbonyl)pyridine-6,4-diyl))bis(ethyne-2,1-diyl))bis(benzene-4,2,1-triyl))tetrakis(oxy))tetraacetate (12)

Compounds **4** (0.162 g, 0.529 mmol) and **11** (0.200 g, 0.221 mmol) were dissolved to a mixture of anhydrous tetrahydrofuran (2.5 ml) and triethylamine (2.5 ml). Bis(triphenylphosphine)palladium(II) dichloride (6.2 mg, 0.009 mmol) and copper (I) iodide (3.4 mg, 0.018 mmol) were added and the mixture was stirred overnight under argon atmosphere at 55°C. The mixture was filtered and concentrated. The product was purified by column chromatography using silica gel as the stationary phase and 10% ethanol :dichloromethane as eluent. Yield: 86.4 mg (29%) MS(ESI-TOF) calculated for C₇₃H₇₈N₆O₂₀ [M⁺] 1358.53, Found: 1358.48

### Example 10. Synthesis of 6,6'-((((4-((4-aminophenyl)ethynyl)pyridine-2,6-diyl)bis(methylene))bis((carboxylatomethyl)azanediyl))bis(methylene))bis(4-((2,4-bis(carboxymethoxy)phenyl)ethynyl)picolinate europium(III) (13)

Compound **12** (84.1 mg, 0.0619 mmol) was dissolved in the mixture of water (2.5 ml) and 0.5M potassium hydroxide solution in ethanol (4.2 ml). Mixture was stirred at RT for 2 h. Reaction mixture was evaporated to dryness, the residue was dissolved in water (2 ml) and pH was adjusted to 6.7 by addition of hydrochloric acid (6M). Europium chloride (22.7 mg, 0.0619 mmol) dissolved in water (0.5 ml) was added to the reaction mixture drop-wise. Reaction mixture was stirred at RT for 1 h 15 min and the pH was maintained at 6.3-6.5 by addition of sodium hydrogen carbonate to the mixture. Excess of europium was precipitated from the mixture by adjusting pH to 8.5-9.0 by addition of sodium hydroxide and the precipitate was removed by centrifugation. The product was precipitated from the supernatant with acetone and isolated by centrifugation. The precipitate was washed with acetone (3x10ml) and the product was dried overnight in vacuum desiccator over silica gel. Yield: 199.4 mg MS(ESI-TOF) calculated for C₅₇H₄₂EuN₆O₂₀⁻ [M-H]²⁻ 641.09, found: 641.16.

### Example 11. Synthesis of 6,6'-((((4-((4-isothiocyanatophenyl)ethynyl)pyridine-2,6-diyl)bis(methylene))bis((carboxylatomethyl)azanediyl))bis(methylene))bis(4-((2,4-bis(carboxymethoxy)phenyl)ethynyl)picolinate) europium(III) (14)

Aqueous solution of compound **13** (90.5 mg, 0.0708 mmol) was added drop-wise in 15 minutes to a mixture of chloroform (1.5ml), thiophosgen (35.8 µl, 0.469 mmol) and sodium hydrogen carbonate (44.4 mg, 0.591 mmol). The two phases were separated and the aqueous phase was washed with chloroform (3x6 ml). The product was precipitated from the aqueous phase with acetone (60 ml) and isolated by centrifugation. The precipitate was washed with acetone (3x10 ml) and dried overnight in vacuum desiccator over silica gel. Yield: 85.0 mg. MS(ESI-TOF) calculated for C₅₈H₄₀EuN₆O₂₀S⁻ [M-H]²⁻ 662.06, found: 662.16.

### Example 12. Synthesis of diethyl 2,2'-(((4-bromo-6-(bromomethyl)pyridin-2-yl)methyl)azanediyl)diacetate (16)

Compound **15** (9.79 g, 28.5 mmol, Acta Chem. Scand B 42, 1988, 373) was dissolved in acetonitrile (196 ml, dry). Diethyl iminodiacetate (2.55ml, 14.2 mmol) was added and the mixture was stirred at 60-65°C for 2 h 30min. The mixture was evaporated to dryness and purified by column chromatography using silica gel as the stationary phase and 20% ethyl acetate:petroleum ether as eluent. Yield 3.36 g (52%). ¹H NMR (CDCl₃, δ ppm): 7.80 (1H, d, J=1.5 Hz), 7.51 (1H, d, 1.5 Hz), 4.47 (2H, s), 4.18 (4H, m), 4.05 (2H, s), 3.62 (4H, s), 1.28 (6H, m).

### Example 13. Synthesis of tetraethyl 2,2',2",2"'-(((6,6'-(((4-aminophenethyl)azanediyl)bis(methylene))bis(4-bromopyridine-6,2-diyl))bis(methylene))bis(azanetriyl))tetraacetate (17)

Compound **16** (3.30 g, 7.29 mmol) was dissolved in acetonitrile (66 ml, dry). Potassium carbonate (5.04 g, 36.5 mmol, dry) and 2-(4-Aminophenyl)ethylamine (0.50 g, 3.68 mmol) were added and the mixture was stirred at 50-55°C for 4 h. The mixture was filtered through celite and the precipitate was washed with acetonitrile. The filtrate was evaporated to dryness and then purified by column chromatography using silica gel as the stationary phase and triethylamine:ethyl acetate:petroleum ether (1:2:7) as eluent. Yield 2.02 g (63%). ¹H NMR (CDCl₃, δ ppm): 7.69 (2H, d, J=1.6 Hz), 7.42 (2H, d, 1.6 Hz), 6.90 (2H, d, J=8.35 Hz), 6.62 (2H, d, J=8.35), 4.17 (8H, m), 4.01 (4H, s), 3.79 (4H, s), 3.60 (8H, s), 2.73 (4H, m), 1.27 (12H, t, J=7.15 Hz).

### Example 14. Synthesis of tetraethyl 2,2',2",2"'-(((6,6'-(((4-aminophenethyl)azanediyl)bis(methylene))bis(4-((2,4-bis(2-ethoxy-2-oxoethoxy)phenyl)ethynyl)pyridine-6,2-diyl))bis(methylene))bis(azanetriyl))tetraacetate (18)

Compounds **4** (0.174 g, 0.568 mmol) and **17** (0.315 g, 0.358 mmol) were dissolved to a mixture of anhydrous tetrahydrofuran (4.0 ml) and triethylamine (4.0 ml). Bis(triphenylphosphine)palladium(II) dichloride (6.6 mg, 0.009 mmol) and copper (I) iodide (3.6 mg, 0.019 mmol) were added and the mixture was stirred overnight under argon atmosphere at 55°C. The mixture was filtered and concentrated. The product was purified by column chromatography using silica gel as the stationary phase and triethylamine:ethyl acetate:petroleum ether (2:3:5) as eluent. Yield: 104.7 mg (22%). ¹H NMR (CDCl₃, δ ppm): 7.52 (2H, s), 7.44 (2H, s), 7.41 (2H, d, J=9.1 Hz), 6.89 (2H, d, 8.3 Hz), 6.58 (2H, d, J=8.3 Hz), 6.40 (4H, m), 4.69 (4H, s), 4.59 (4H, s), 4.30-4.13 (16H, m), 4.04 (4H, s), 3.86 (4H, s), 3.61 (8H, s), 2.76 (4H, m), 1.32-1.23 (24H, m).

### Example 15. Synthesis of 2,2',2",2"'-(((6,6'-(((4-aminophenethyl)azanediyl)bis-(methylene))bis(4-((2,4-bis(carboxymethoxy)phenyl)ethynyl)pyridine-6,2-diyl))bis-(methylene))bis(azanetriyl))tetraacetate europium(III) (19)

Compound **18** (101.2 mg, 0.076 mmol) was dissolved in the mixture of water (3.3 ml) and 0.5M potassium hydroxide solution in ethanol (5.1 ml). Mixture was stirred at RT for 2 h. Reaction mixture was evaporated to dryness and the residue was dissolved in water (1.7 ml). The solution was stirred for 30 min and the pH was adjusted to 6.7 by addition of hydrochloric acid (6M). Europium chloride (27.9 mg, 0.076 mmol) dissolved in water (0.6 ml) was added to the reaction mixture drop-wise. Reaction mixture was stirred at RT for 45 min and the pH was maintained at 6.3-6.7 by addition of sodium hydrogen carbonate to the mixture. Excess of europium was precipitated from the mixture by adjusting pH to 8.5-9.0 by addition of sodium hydroxide and the precipitate was removed by centrifugation. The product was precipitated from the supernatant with acetone and isolated by centrifugation. The precipitate was washed with acetone (3x10ml) and the product was dried overnight in a vacuum desiccator over silica gel. Yield: 279.4 mg. MS(ESI-TOF) calculated for C₅₄H₄₈EuN₆O₂₀⁻ [M-H]²⁻ 626.10, found: 626.16.

### Example 16. Synthesis of 2,2',2",2"'-(((6,6'-(((4-isothiocyanatophenethyl)azanediyl)-bis(methylene))bis(4-((2,4-bis(carboxymethoxy)phenyl)ethynyl)pyridine-6,2-diyl))bis-(methylene))bis(azanetriyl))tetraacetate europium(III) (20)

Aqueous solution of compound **19** (99.8 mg, 0.0797 mmol) was added drop-wise in 10 minutes to a mixture of chloroform (1.6ml), thiophosgen (40.4 µl, 0.530 mmol) and sodium hydrogen carbonate (50.2 mg, 0.597 mmol). Mixture was stirred at RT for 20 min. Phases were separated and the aqueous phase was washed with chloroform (4x2 ml). Product was precipitated from the aqueous phase with acetone (60 ml) and isolated by centrifugation. The precipitate was washed with acetone (3x30 ml) and dried overnight in a vacuum desiccator over silica gel. Yield: 116.2 mg. MS(ESI-TOF) calculated for C₅₅H₄₆EuN₆O₂₀S⁻ [M-H]²⁻ 647.08, found: 647.15.

### Example 17. Synthesis of triethyl 2,2',2"-((4-iodo-1,3,5-phenylene)tris(oxy))triacetate (21)

A mixture of 4-Iodo-1,3,5-trihydroxybenzene (3.41 g, 13.5 mmol; Acta Chem. Scand. 1991, 45, 539), dry K₂CO₃ (6.17 g, 44.7 mmol), ethyl bromoacetate (4.96 ml, 44.7 mmol) and dry MeCN (100 ml) was stirred overnight at 55°C. The mixture was filtered, the solid material washed with MeCN and the filtrate was evaporated to dryness. The product was purified by column chromatography using silica gel as stationary phase and MeOH:CH₂Cl₂ (first 0:100, then 20:80) as eluent. Yield: 0.64 g (9%). ¹H NMR (CDCl₃, δ ppm): 6.10 (2H, s), 4.65 (4H, s), 4.55 (4H, s), 4.27 (2H, q, J=7.15 Hz), 4.26 (4H, q, J=7.15 Hz), 1.31 (3H, t, J=7.15 Hz), 1.30 (6H, t, J=7.15 Hz). MS(ESI-TOF) calculated for C₁₈H₂₃IO₉ [M+H]⁺: 511.05, found: 510.93.

### Example 18. Synthesis of triethyl 2,2',2"-((4-(trimethylsilyl)ethynyl-1,3,5-phenylene)tris(oxy))-triacetate (22)

This compound **22** was synthesized from the compound **21** using a method analogous to the synthesis described in the Example 2. The mixture was stirred first at 100°C for 30 minutes, then at 120°C for 20 minutes by using microwave heating. The mixture was extracted with Et₂O (50 ml), washed with H₂O (2 x 20 ml), dried with Na₂SO₄, and the product purified by column chromatography using silica gel as stationary phase and ethyl acetate:petroleum ether (first 20:80, then 30:70) as eluent. Yield: 70%. ¹H NMR (CDCl₃, δ ppm): 6.10 (2H, s), 4.67 (4H, s), 4.55 (2H, s), 4.27 (2H, g, J=7.15 Hz), 4.26 (4H, q, J=7.15 Hz), 1.30 (6H, t, J=7.15 Hz), 1.29 (3H, t, J=7.15 Hz), 0.26 (9H, s). MS(ESI-TOF) calculated for C₂₃H₃₂O₉Si [M+H]⁺: 481.19, found: 481.99.

### Example 19. Synthesis of triethyl 2,2',2"-((4-ethynyl-1,3,5-phenylene)tris(oxy))triacetate (23)

This compound **23** was synthesized from the compound **22** using a method analogous to the synthesis described in the Example 3. After washings with 10% citric acid and H₂O, the product (100%) was used for the next step without further purifications. ¹H NMR (CDCl₃, δ ppm): 6.06 (2H,s), 4.69 (4H, s), 4.55 (2H, s), 4.27 (2H, q, J=7.15 Hz), 4.26 (4H, q, J=7.15 Hz), 3.50 (1H, s), 1.30 (3H, t, J=7.15 Hz), 1.29 (6H, t, J=7.15 Hz). MS(ESI-TOF) calculated for C₂₀H₂₄O₉ [M+H]⁺: 409.15, found: 409.20.

### Example 20. Synthesis of N-(4-ethynylphenyl)-2,2,2-trifluoroacetamide (24)

4-Ethynylaniline (1.38 g, 11.8 mmol) was added in small portions into an ice-cold (CF₃CO)₂O (6.6 ml). After stirring for 10 min in ice-bath, the mixture was stirred for 2.5 hours at room temperature. The mixture was poured into ice-H₂O (100 ml), the product filtered and washed with H₂O. The product (2.29 g, 91%) was used for the next step without further purifications. ¹H NMR (CDCl3): δ (ppm)) 8.08 (1H, s), 7.55 (2H, d, J=8.80 Hz), 7.51 (2 H, d, J=8.80 Hz), 3.1 (1H, s). MS(ESI-TOF) calculated for C₁₀H₆F₃NO [M+H]⁺: calculated 214.02, found 213.07.

### Example 21. Synthesis of N-(4-((2,6-bis(hydroxymethyl)pyridin-4-yl)ethynyl)phenyl)-2,2,2-trifluoroacetamide (25)

A mixture of the compound **24** (0.55 g, 2.58 mmol) and 6-bromo-2,6-dihydroxymethylpyridine (0.47 g, 2.15 mmol; Acta Chem. Scand. 1988, Ser B, 42, 614) in dry triethylamine (5 ml) and tetrahydrofurane (10 ml) was de-aerated with argon. After addition of bis(triphenylphosphine)-palladium(II) chloride (30 mg, 43 µmol) and CuI (16 mg, 86 µmol), the mixture was stirred for 19 hours at 55°C. After evaporation to dryness, the residue was treated with a cold mixture of CH₂Cl₂ (40 ml) and H₂O (20 ml), filtered and the product (0.56 g, 75%) washed with cold H₂O (10 ml) and CH₂Cl₂ (10 ml). Yield: 0.56 g (75%). ¹H NMR (D6-DMSO, δ ppm): 11.4 (1H, bs), 7.79 (2H, d, J=8.80 Hz), 7.68 (2H, d, J=8.80 Hz), 7.42 (2H, s), 5.49 (2H, bs), 4.56 (4H, s). MS(ESI-TOF) calculated for C₁₇H₁₃F₃N₂O₃ [M+H]⁺: calculated 351.10, found 351.96.

### Example 22. Synthesis of N-(4-((2,6-bis(bromomethyl)pyridin-4-yl)ethynyl)phenyl)-2,2,2-trifluoroacetamide (26)

PBr₃ (225 µl) was added in a suspension of the compound **24** (0.56 g, 1.6 mmol) in CHCl₃ (65 ml). After stirring for 20 hours at 60°C, the mixture was neutralized with 5% NaHCO₃ (35 ml). The aqueous phase was extracted with CHCl₃ (40 ml) and the combined organic phases were dried with Na₂SO₄, filtered and evaporated to dryness. The product **25** (1.09 g, 93%) was used for the next step without further purifications. ¹H NMR (CDCl₃, δ ppm): 7.93 (1H, s), 7.63 (2H, d, J=8.68 Hz), 7.59 (2H, J=8.68 Hz), 7.47 (2H, s), 4.53 (4H, s). MS(ESI-TOF) calculated for C₁₇H₁₁Br₂F₃N₂O [M+H]⁺: calculated 474.93, 476.93, 498.92, found 475.38, 477.44, 479.45.

### Example 23. Synthesis of diethyl 6,6'-((((4-((4-trifluoroacetamidophenyl)ethynyl)-pyridine-2,6-diyl)bis(methylene))bis((2-ethoxy-2-oxoethyl)azanediyl))bis(methylene))-bis(4-bromopicolinate) (27)

This compound **27** was synthesized from the compounds **26** and **9** using a method analogous to the synthesis described in the Example 7. Reaction time at 70 °C was 27 hours. The product was purified by column chromatography using silica gel as stationary phase and triethylamine:ethyl acetate:petroleum ether (1:69:30) as eluent. Yield: 66%. ¹H NMR (CDCl₃, δ ppm): 8.53 (1H, s), 8.14 (2H, d, J=1.80 Hz), 8.11 (2H, d, J=1.80 Hz), 7.68 (2 H, d, J=8.73 Hz), 7.60 (2H, d, J=8.73 Hz), 7.34 (2H, s), 4.44 (4H, q, J=7.08 Hz), 4.18 (4 H, q, J=7.15 Hz); 4.10 (4H, s), 3.98 (4 H, s), 3.50 (4 H, s), 1.40 (6H, t, J=7.08 Hz), 1.28 (6H, t, J=7.15 Hz). MS(ESI-TOF) calculated for C₄₃H₄₃Br₂F₃N₆O₉ [M+H]⁺: calculated 1003.15, 1005.15, 1007.15, found 1003.71, 1005.48, 1007.58.

### Example 24. Synthesis of the compound 28

A mixture of the compound **27** (120 g, 0.148 mmol) and **23** (145 mg, 0.355 mmol) in dry triethylamine (1 ml) and tetrahydrofurane (2 ml) was de-aerated with argon. After addition of bis(triphenylphosphine)palladium(II) chloride (10 mg, 14 µmol) and CuI (6 mg, 28 µmol), the mixture was stirred for 22 hours at 55°C. After evaporation to dryness, the residue was dissolved in CH₂Cl₂ (40 ml), washed with H₂O (3 x 10 ml) and dried with Na₂SO₄. The product was purified by column chromatography using silica gel as stationary phase and triethylamine:ethyl acetate:petroleum ether (1:69:30) as eluent. Yield: 115 mg (47%). ¹H NMR (CDCl₃, δ ppm): 9.12 (1H, s), 8.09 (2H, d, J=1.25 Hz), 8.06 (2H, d, J=1.25 Hz), 7.57 (2 H, s), 7.34 (2H, d, J=8.80 Hz), 7.30 (2H, d, J=8.80 Hz), 5.97 (4H, s), 4.63 (8H, s), 4.60 (4H, s), 4.45 (4H, q, J=7.12 Hz), 4.30 (4H, q, J=7.12 Hz), 4,21 (8H, q, J=7.12 Hz), 4.17 (4H, q, J=7.12), 4.10 (4H, s), 4.02 (4H, s), 3.52 (4H, s), 1.42 (6H, t, J=7.12 Hz), 1.33 (6H, t, J=7.12 Hz), 1.29 (6H, J=7.12 Hz), 1.25 (12H, t, J=7.12 Hz). MS(ESI-TOF) calculated for C₈₃H₈₉F₃N₆O₂₇ [M+H]⁺: calculated 1659.58, found 1659.81.

### Example 25. Synthesis of the europium(III) chelate 29

A mixture of the compound **28** (105 mg, 63 µmol) and 0.5M KOH in ethanol (9 ml) was stirred for 30 minutes at room temperature and water was added (2 ml). After stirring for 3 hours at room temperature, EtOH was evaporated, the residue was stirred for 30 minutes at room temperature, and the pH was adjusted to ca. 6.5 with 6M HCl. Europium(III) chloride (23 mg, 63 µmol) in H₂O (0.17 ml) was added within 10 minutes and the pH was maintained at 5-7 with solid NaHCO₃. After stirring for overnight at room temperature, the pH was raised to 8.5 with 1M NaOH, the precipitate was centrifuged off and the supernatant was extracted with phenol (once with 0.75 g and 3 x 0.5 g). The combined phenol phases were treated with H₂O (1 ml) and Et₂O (20 ml), the aqueous phase was washed with Et₂O (2 x 20 ml), and triturated with acetone. The precipitate was centrifuged and washed with acetone. The product was used for the next step without further purification. Conditions for HPLC run: Reversed phase HPLC (RP-18 column). The solvents were A: Triethyl ammonium acetate buffer (20mM, pH7) and B: 50% acetonitrile in triethyl ammonium acetate buffer (20mM, pH7). The gradient was started from 5% of solvent B and the amount of solvent B was linearly raised to 100 % in 25 minutes. R_{f}(HPLC)=14.5 min. UV/VIS=359 nm.

### Example 26. Synthesis of the europium(III) labeling chelate 30

This compound **30** was synthesized from the chelate **29** using a method analogous to the synthesis described in the Example 11. Conditions for HPLC run: see example 35. R_{f}(HPLC)=20.9 min. UV/VIS=325 (sh), 340 and 362 (sh) nm.

### Example 27. Synthesis of the europium(III) chelate 31

This compound **31** was synthesized from the chelate **30** using a method analogous to the synthesis described in the Example 38. R_{f}(HPLC)=14.3 min. UV/VIS=349 nm.

### Example 28. Synthesis of 1-Bromo-3,5-dihydroxybenzene (33)

1-bromo-3,5-dimethoxybenzene (**32**) (1.00 g, 4.60 mmol) was dissolved in dry dichloromethane (40ml) and cooled in an ice bath. Boron tribromide (1.33 ml, 13.82 mmol) was added and the mixture was stirred on 2 h. The mixture was allowed to warm up to room temperature and stirred overnight. Methanol (1.4 ml) was added drop-wise to terminate the reaction, and the mixture was poured into water (50 ml) and stirred at RT for 2 h. Reaction mixture was neutralized with NaHCO₃ and the mixture extracted twice with ethyl acetate (30 ml). Combined organic layers were dried over Na₂SO₄ and evaporated to dryness. Product was purified by column chromatography using silica gel as stationary phase and methanol:dichloromethane (5:95) as eluent. Product was a white solid. Yield: 0.71 g (81%). ¹HNMR (DMSO-d6, δ ppm): 9.68 (2H, s), 6.38 (2H, d, J = 2 Hz), 6.19 (1H, dd, J = 2 Hz).

### Example 29. Synthesis of di-tert-butyl 2,2'-((5-bromo-1,3-phenylene)bis(oxy))diacetate (34)

Compound **33** (0.49 g, 2.59 mmol) was dissolved in DMF (10 ml, dry). Anhydrous K₂CO₃ (2.15 g, 15.56 mmol) and *tert*-butyl bromoacetate (1.15 ml, 7.78 mmol) were added and the mixture was stirred overnight under argon atmosphere at 50°C. Water (17 ml) was added and the mixture was extracted with ethyl acetate (3x40 ml). Combined organic extracts were dried over NaHCO₃ and evaporated to dryness. Crude product was purified by column chromatography using silica gel as stationary phase and dichloromethane as eluent. Product was a white solid. Yield: 0.95 g (87%) ¹H NMR (CDCl₃, δ ppm): 6.67 (2H, d, J = 2 Hz), 6.42 (1H, dd, J = 2 Hz), 1.49 (18H, s)

### Example 30. Synthesis of di-tert-butyl 2,2'-((5-((trimethylsilyl)ethynyl)-1,3-phenylene)bis(oxy))diacetate (35)

Compound **34** (2.00 g, 4.79 mmol) was dissolved in DMF (4 ml, dry) and the solution was placed in a microwave reaction vial. Diethyl amine (12 ml, dry), Pd(PPh₃)₂Cl₂ (168 mg, 0.24 mmol), CuI (46 mg, 0.24 mmol) and PPh₃ (251 mg, 0.96 mmol) were added and the vial was sealed in an argon atmosphere. Trimethylsilyl acetylene (996 µl, 7.19 mmol) was added through a septum and the mixture was stirred at 120°C for 30 minutes using microwave heating. Reaction mixture was evaporated to dryness, dissolved in dichloromethane and purified by column chromatography using silica gel as stationary phase and ethyl acetate:petroleum ether (10:90) as eluent. Yield: 1.36 g (65%) ¹H NMR (CDCl₃, δ ppm): 6.61 (2H, d, J = 2.35 Hz), 6.49 (1H, dd, J = 2.3 Hz), 4.46 (4H, s), 1.49 (18H, s), 0.23 (9H, m).

### Example 31. Synthesis of di-tert-butyl 2,2'-((5-ethynyl-1,3-phenylene)bis(oxy))diacetate (36)

Compound **35** (1.29 g, 2.98 mmol) was dissolved in dichloromethane (40 ml, dry). Tetrabutyl ammonium fluoride (0.934 g, 3.57 mmol) was added and the mixture was stirred in argon atmosphere at room temperature for 45 min. Mixture was washed with 10% citric acid solution (20 ml), and four times with water (4x40 ml). The organic phase was dried over Na₂SO₄ and evaporated to dryness. Crude product was purified by column chromatography using silica gel as stationary phase and ethyl acetate:petroleum ether (10:90) as eluent. Product was a yellowish solid. Yield: 895 mg (83%). ¹H NMR (CDCl₃, δ ppm): 6.64 (2H, d, J = 2.3 Hz), 6.51 (1H, dd, J = 2.33 Hz), 4.47 (4H, s), 3.02 (1H, s), 1.49 (18H, s).

### Example 32. Synthesis of tetra-tert-butyl 2,2',2",2"'-((((6,6'-((((4-((4-aminophenyl)ethynyl)pyridine-2,6-diyl)bis(methylene))bis((2-ethoxy-2-oxoethyl)azanediyl))bis(methylene))bis(2-(ethoxycarbonyl)pyridine-6,4-diyl))bis(ethyne-2,1-diyl))bis(benzene-5,3,1-triyl))tetrakis(oxy))tetraacetate (37)

Compound **36** (0.167 g, 0.459 mmol) and compound **11** (0.167 g, 0.184 mmol) were dissolved in mixture of anhydrous tetrahydrofurane (4 ml) and triethylamine (2.5 ml). Bis(triphenylphosphine)palladium(II) dichloride (3.8 mg, 0.0054 mmol) and copper (I) iodide (2.3 mg, 0.012 mmol) were added and the mixture was stirred overnight under argon atmosphere at 55-60°C. Reaction mixture was evaporated to dryness and purified by column chromatography using silica gel as stationary phase and methanol:dichloromethane (10:90) as eluent. Yield: 71 mg (27%) MS(ESI-TOF) calculated for C₈₁H9₄N₆O₂₀ [M+H]⁺: 1471.66, Found: 1471.68

### Example 33. Synthesis of 6,6'-((((4-((4-aminophenyl)ethynyl)pyridine-2,6-diyl)bis(methylene))bis((carboxylatomethyl)azanediyl))bis(methylene))bis(4-((3,5-bis(carboxymethoxy)phenyl)ethynyl)picolinate europium(III) (38)

Compound **37** (68 mg, 0.048 mmol) was dissolved in trifluoroacetic acid (1 ml, 13.5 mmol) and the mixture was stirred at RT for 1.5 h. Reaction mixture was evaporated to dryness without heating. Diethyl ether (2.5ml) was added to the residue and the mixture was stirred for 15 minutes. Product precipitated from the mixture and was isolated by centrifugation. The precipitate was washed twice with diethyl ether and then dried in vacuum desiccator over silica gel. Dried precipitate (64 mg, 0.051 mmol) was dissolved in the mixture of water (1.8 ml) and 0.5M potassium hydroxide solution in ethanol (4 ml). Mixture was stirred in RT for 2 h. Reaction mixture was evaporated to dryness and the residue was dissolved in water (1.7 ml). pH was adjusted to 6.5 by addition of hydrochloric acid (6M). Europium chloride (20 mg, 0.055 mmol) dissolved in water (485 µl) was added to the reaction mixture drop-wise. Reaction mixture was stirred at RT for 2 hours and the pH was maintained at 6.3-6.5 by addition of sodium hydrogen carbonate to the mixture. Excess of europium was precipitated from the mixture by adjusting pH to 8.5-9.0 by addition of sodium hydroxide and the precipitate was removed by centrifugation. The product was precipitated with acetone (25 ml) and isolated by centrifugation. The precipitate was washed with acetone (3x10 ml) and dried overnight in vacuum desiccator over silica gel. Yield: 180 mg. MS(ESI-TOF) calculated for C₅₇H₄₂EuN₆O₂₀⁻ [M+2H]⁺ (for free acids without sodium ions): 1285.18, Found: 1285.24

### Example 34. Synthesis of 6,6'-((((4-((4-isothiocyanatophenyl)ethynyl)pyridine-2,6-diyl)bis(methylene))bis((carboxylatomethyl)azanediyl))bis(methylene))bis(4-((3,5-bis(carboxymethoxy)phenyl)ethynyl)picolinate) europium(III) (39)

Aqueous solution of compound **18** (175 mg, 0.143 mmol) was added drop-wise to a mixture of chloroform (2.85 ml), thiophosgene (74 µl, 0.97 mmol) and sodium hydrogen carbonate (91 mg, 1.09 mmol). Reaction mixture was stirred at RT for 45 min. The two phases were separated and the aqueous phase was washed with chloroform (3x6 ml). pH was adjusted to 7 with 1M acetic acid and the product was precipitated with acetone (57 ml) and isolated by centrifugation. The precipitate was washed with acetone (3x15 ml) and dried overnight in vacuum desiccator over silica gel. Yield: 216 mg. MS(ESI-TOF) calculated for C₅₈H₄₀EuN₆O₂₀S⁻ [M-H]²⁻ (for free acids without sodium ions): 662.56, Found: 662.47.

### Example 35. Synthesis of tetra(tertbutyl) 2,2',2",2"'-(((6,6'-(((4-aminophenethyl)azanediyl)bis(methylene))bis(4-((3,5-bis(2-ethoxy-2-oxoethoxy)phenyl)ethynyl)pyridine-6,2-diyl))bis(methylene))bis(azanetriyl))tetraacetate (40)

Compound **40** was prepared from compounds **36** (0.88 g, 2.44 mmol) and tetra(*tert*-butyl) 2,2',2",2"'-(((6,6'-(((4-aminophenethyl)azanediyl)bis(methylene))bis(4-bromopyridine-6,2-diyl))bis(methylene))bis(azanetriyl))tetraacetate (1.01 g, 1.02 mmol) (prepared according to von Lode P. et al., Anal. Chem., 2003, 75, 3193-3201) using a method analogous to the synthesis described in the Example 14. MS(MALDI) calculated for C₈₆H₁₁₆N₆O₂₀ [M+H]⁺: 1554.82, Found: 1554.71.

### Example 36. Synthesis of 2,2',2",2"'-(((6,6'-(((4-aminophenethyl)azanediyl)bis-(methylene))bis(4-((3,5-bis(carboxymethoxy)phenyl)ethynyl)pyridine-6,2-diyl))bis-(methylene))bis(azanetriyl))tetraacetate europium(III) (41)

Compound **40** (0.87 g, 0.56 mmol) was dissolved in trifluoroacetic acid (20 ml, 250 mmol) and the mixture was stirred in RT for 2 h. Reaction mixture was evaporated to dryness without heating. Diethyl ether (30 ml) was added to the residue and the mixture was stirred for 15 minutes. Precipitated product was isolated by centrifugation. The precipitate was washed with diethyl ether and then dried in vacuum desiccator over silica gel. Dried precipitate (0.30 g) was dissolved in water (6 ml). pH was adjusted to 6.5 by addition of sodium hydroxide and hydrochloric acid. Europium chloride (0.10 mg, 0.27 mmol) in water (2 ml) was added to the reaction mixture drop-wise. pH was maintained at 6.3-6.5 by addition of solid sodium hydrogen carbonate to the mixture. Reaction mixture was stirred at RT for 30 minutes. pH was adjusted to 8.5-9.0 by addition of sodium hydroxide. The product was precipitated with acetone and isolated by centrifugation. The product was dried overnight in vacuum desiccator over silica gel. MS(ESI-TOF) calculated for C54H48EuN6O20- [M+3H]2+: 628.01, Found: 628.12. HPLC retention time 15.38 minutes (buffers and conditions same as in example 35). UV-maximum 304 nm.

### Example 37. Synthesis of 2,2',2",2"'-(((6,6'-(((4-isothiocyanatophenethyl)azanediyl)-bis(methylene))bis(4-((3,5-bis(carboxymethoxy)phenyl)ethynyl)pyridine-6,2-diyl))bis-(methylene))bis(azanetriyl))tetraacetate europium(III) (42)

Compound **42** was prepared from **41** using a method analogous to the synthesis described in the Example 16. MS(ESI-TOF) calculated for C55H46EuN6020S- [M+H]: 1294.17, Found: 1294.72. HPLC retention time 17.42 minutes (buffers and conditions same as in Example 38). UV-maximum 302 nm.

### Example 38. Preparation of taurine derivative 43, 44 and 46

Compound **14** (10.0 mg), compound **20** (10.0 mg) and conventional 9-dentate α-galactose Eu chelate (**45**) (prepared according to von Lode P. et al., Anal. Chem., 2003, 75, 3193-3201) (5.5 mg) respectively were coupled to taurine (5 mg for each reaction) by dissolving the starting materials in aqueous sodium hydrogen carbonate (2 ml, 50 mM, pH9.8). The mixtures were incubated overnight at RT. The products (**43, 44** and **46**) were purified by using reversed phase HPLC (RP-18 column). The solvents were A: Triethyl ammonium acetate buffer (20 mM, pH7) and B: 50% acetonitrile in triethyl ammonium acetate buffer (20 mM, pH7). The gradient was started from 5% of solvent B and the amount of solvent B was linearly raised to 100 % in 30 minutes except for **46**, for which it was raised to 100% in 25 minutes. The products eluted from the column at 16.9 min (**43**), 15.1 (**44**) and at 15.1 min (**46**) time points. Fractions containing the products were collected, pooled and evaporated to dryness.

The other two taurine derivatives **47** and **48** were prepared from corresponding chelates **39** and **42**, respectively, using the same method described above. The products eluted from the column at 16.6 min (**47**) and at 14.5 min (**48**) time points.

### Example 39. Excitation spectrum of compound 20

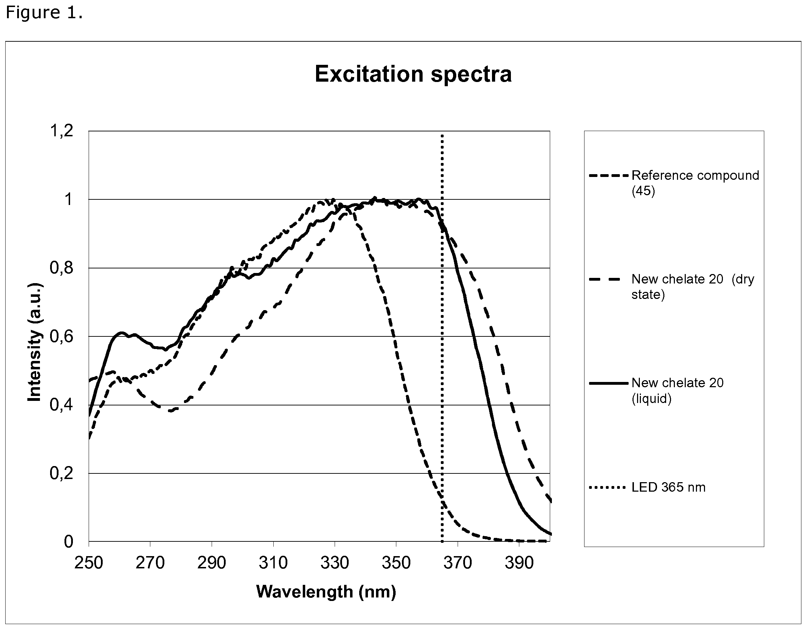

The excitation spectrum of the prepared chelate **20** was measured both in liquid phase and in dry state. For comparison also an excitation spectrum of the reference compound (**45**) is presented in Figure 1. (liquid phase measurement, dashed line).

The excitation spectrum shows a clear red-shift compared to the reference compound in the excitation maximum thus allowing efficient excitation also with longer wavelength for example excitation with 365 nm LED.

### Example 40. Comparison between luminescence of taurine derivatives of Eu-chelates according to present invention (43 (taurine of compound 14) and 44 (taurine of compound 20) and a conventional 9-dentate α-galactose Eu chelate (46 (taurine of compound 45))

The purified taurine derivative were dissolved in water and analyzed for UV spectrum, Eu-content with DELFIA® against Eu standard material and luminescence signal in TSA buffer for **43** and **44**, and in water for **46** with Victor™ Plate fluorometer. Luminescence yields were calculated using the luminescence measurement data and luminescence yield reported by von Lode P. et al., Anal. Chem., 2003, 75, 3193-3201. The results are summarized in Table 1.

**Table 1.**

| | **Compound 46 (reference compound)** | **Compound 43** | **Compound 44** |
|---|---|---|---|
| Molar absorptivity (ε) based on Eu content | 56,000 | 85,000 | 59,000 |
| Luminescence yield (εΦ) | 4,800 | 14,900 | 5,500 |

### Example 41. Comparison between luminescence properties of Eu-chelates (43 and 44) according to present invention () and conventional chelates 47 and 48, respectively.

The measured photo-physical properties excitation wavelengths (λ_{exc}), luminescence decay times (τ), molar absorptivities (ε), estimated luminescence yields (εΦ) of the novel chelates **43** and **44** compared to the conventional chelates **47** and **48**, respectively, in 50 mM TRIS buffer (pH 7.75) are in the Table 2 and 3.

**Table 2.**

| Chelate | ε | εϕ | ϕ | Mean lifetime (ms) | Excitation max. (nm) |
|---|---|---|---|---|---|
| **47** | 83000 | 14100 | 0.17 | 0.93 | 323 |
| **43** | 85000 | 14900 | 0.18 | 0.84 | 343 |

**Table 3.**

| | ε | εϕ | ϕ | excitation max. (nm) |
|---|---|---|---|---|
| **44** | 57000 | 8500 | 0.15 | 340 |
| **48** | 59000 | 3300 | 0.06 | 320 |

### Example 42. Labeling of antibody with compound 14 and 39

Labeling of an TnI antibody was performed as described in von Lode P. et al., Anal. Chem., 2003, 75, 3193-3201 by using a 90 fold excess of the compounds **14** and **39.** The reaction was carried out overnight at RT. Labeled antibody was separated from the excess of the chelate on Superdex 200 HR 10/30 gel filtration column (GE healthcare) by using Tris-saline-azide (Tris 50 mM, NaCl 0.9%, pH 7.75) buffer as an eluent. The fractions containing the antibody were pooled and the europium concentration was measured against Eu standard material with Victor™ plate-fluorometer. The labelling degree of ∼2.6 labels/antibody was obtained.

The measured photo-physical properties excitation wavelengths λ_{exc}), luminescence decay times (τ), molar absorptivities (ε), luminescence yields (εΦ) of the labelled cTnIs with the chelates **14** compared to the chelate **39** in 50 mM TRIS buffer (pH 7.75) in the Table 4.

Dry measurements (**14** (dry) and **39** (dry)) represents estimated luminescence yields based on the signal measurements after dry immunoassay done as described in the literature (von Lode P. et al., Anal. Chem., 2003, 75, 3193-3201).

**Table 4.**

| Labeled IgG with chelate | ε | εΦ | Φ | mean lifetime (ms) | excitation max. (nm) |
|---|---|---|---|---|---|
| **39** | 70000 | 9100 | 0.13 | 0.90 | 323 |
| dry | | 10200 | | | |
| **14** | 77000 | 16100 | 0.21 | 0.80 | 343 |
| dry | | 14500 | | | |

### Example 43. Excitation spectrum of labeled antibody

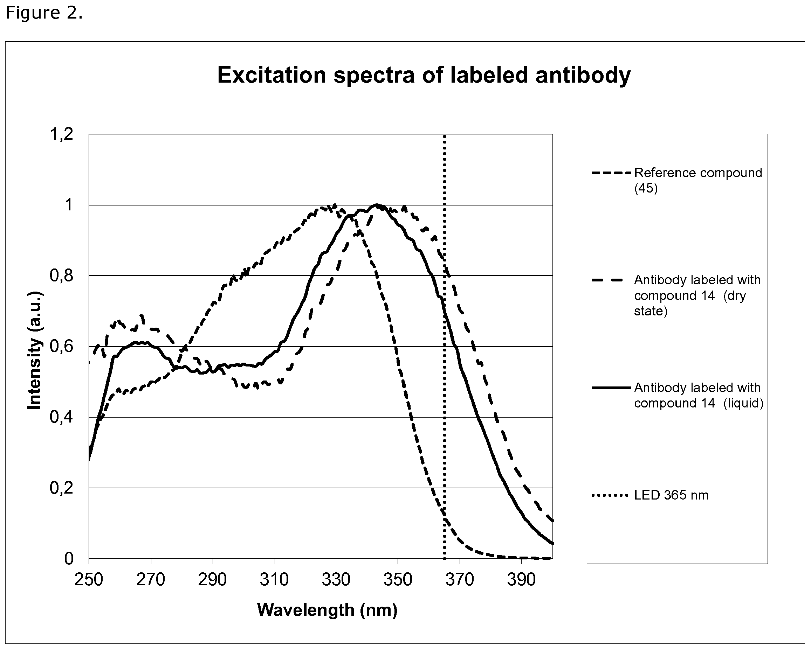

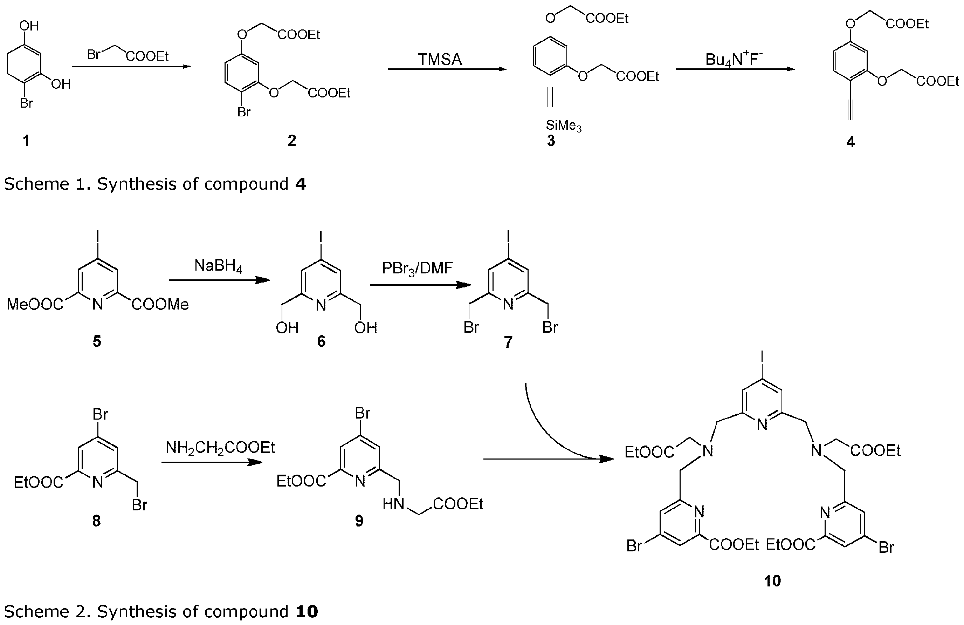

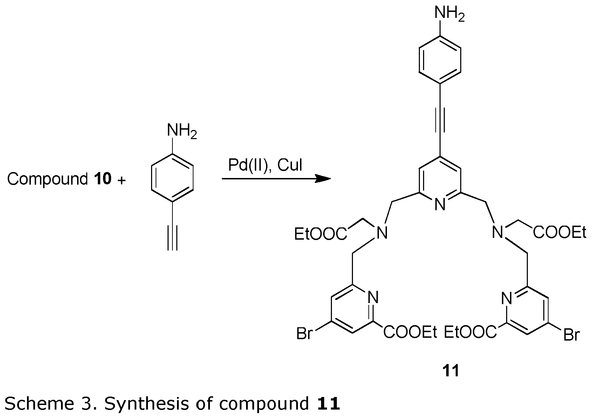

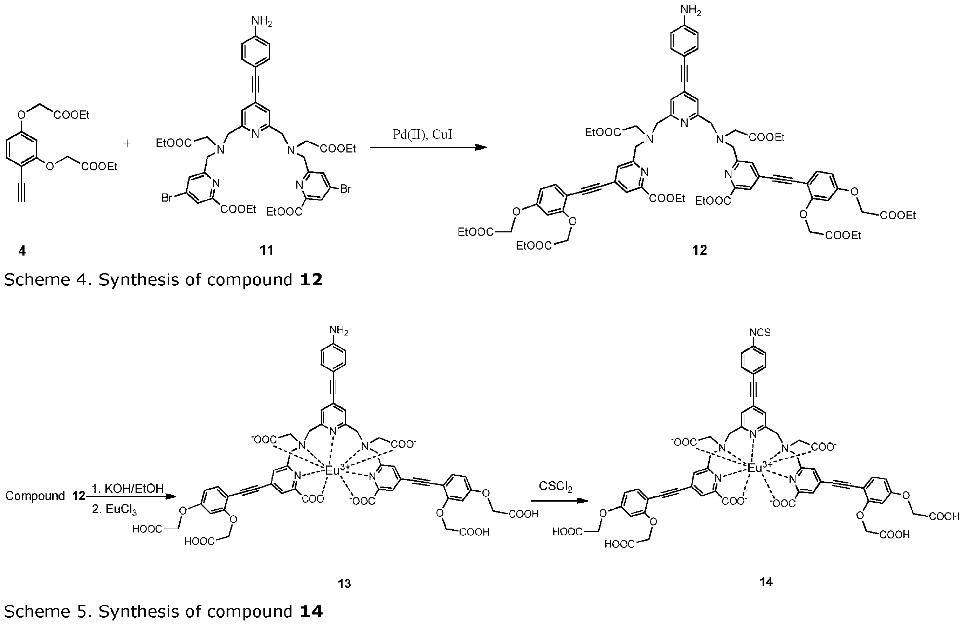

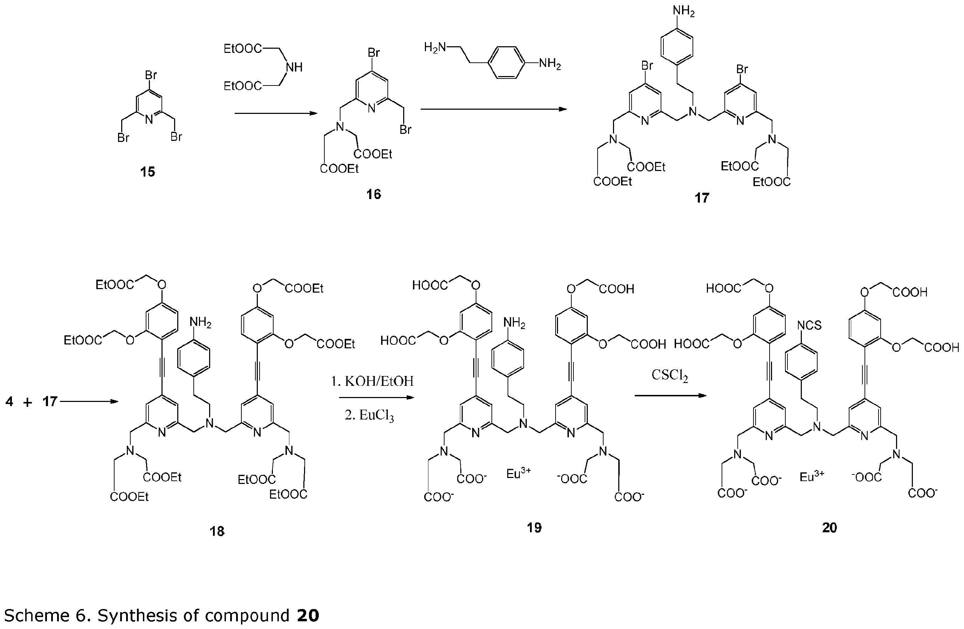

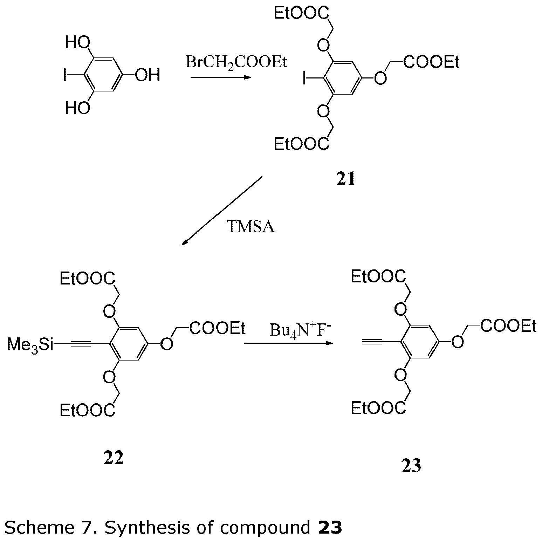

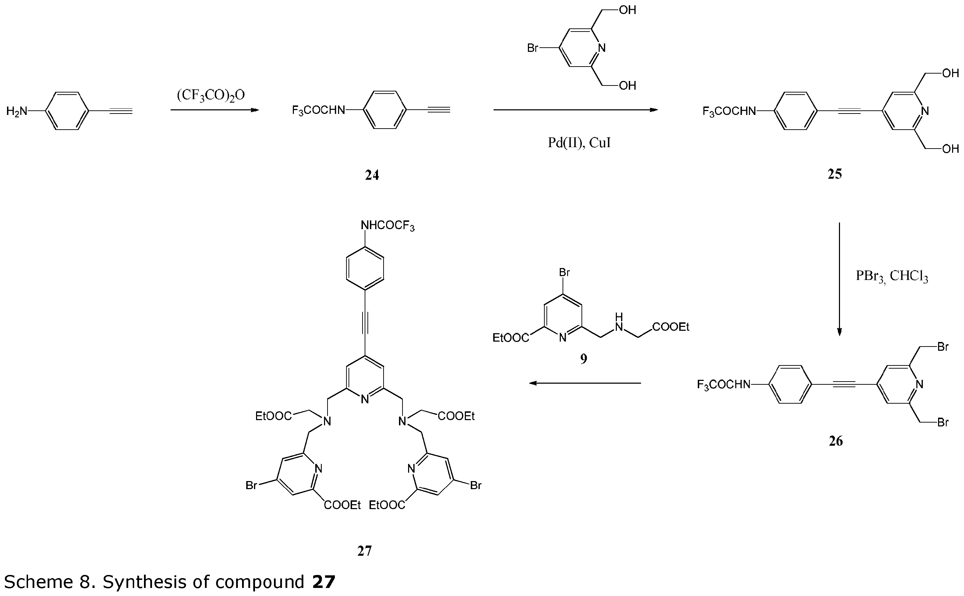

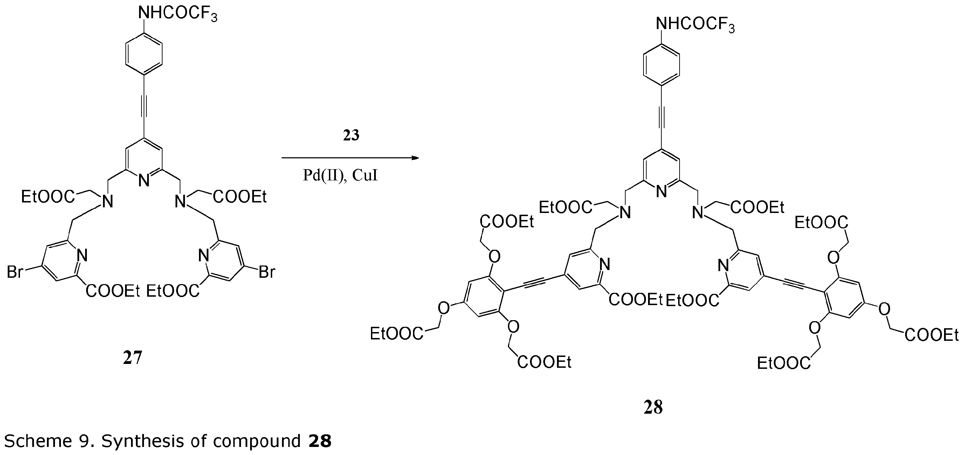

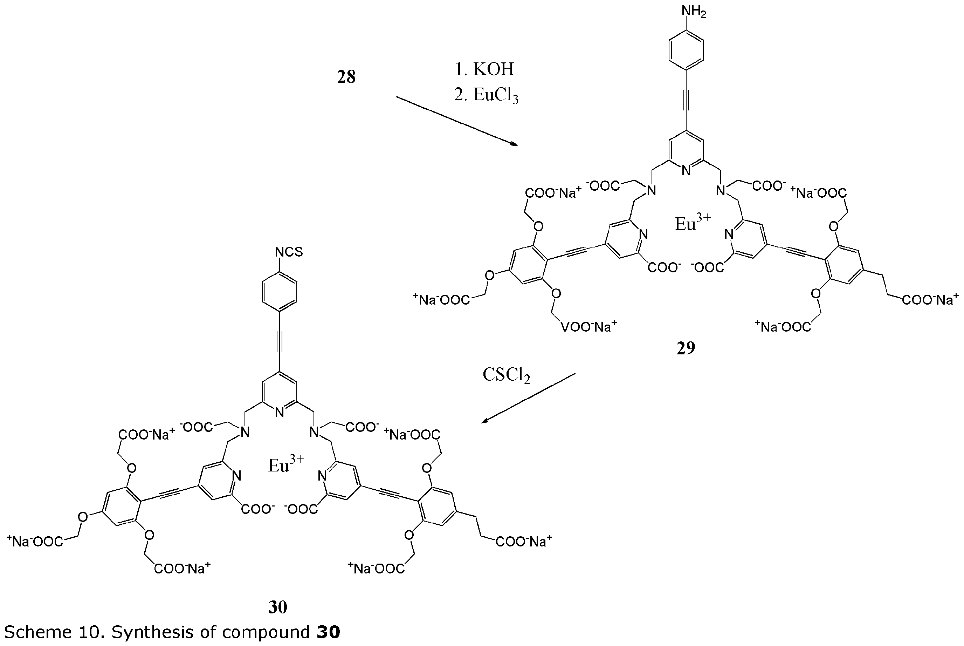

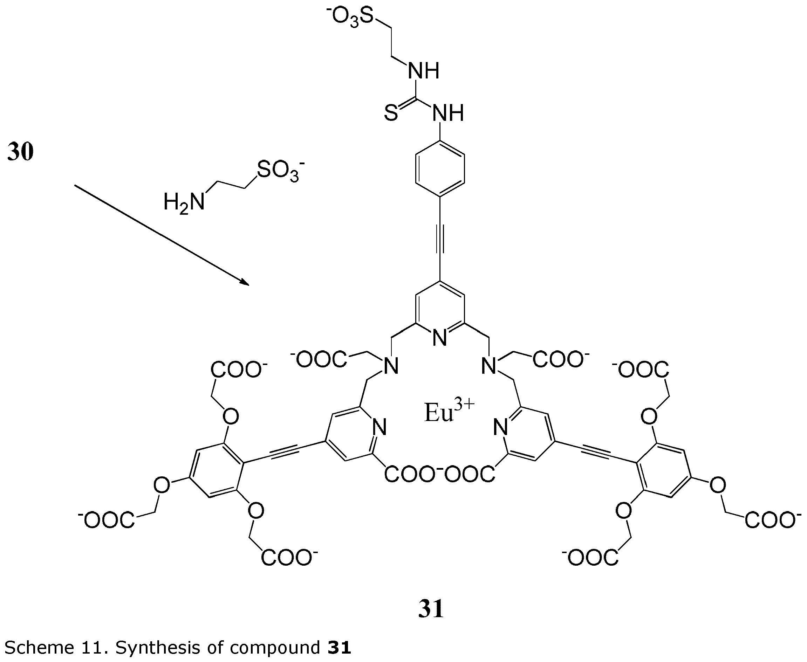

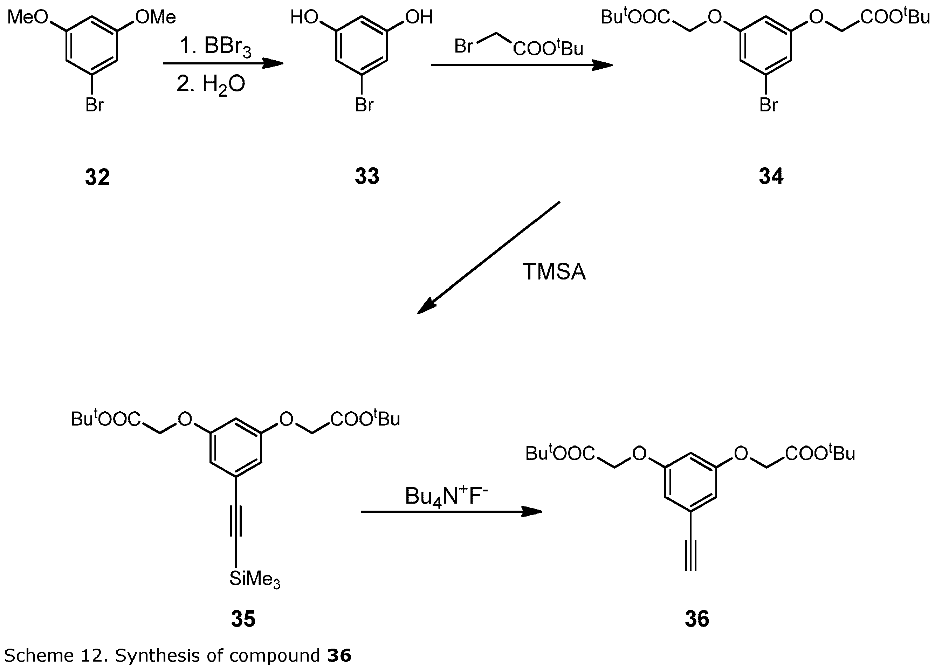

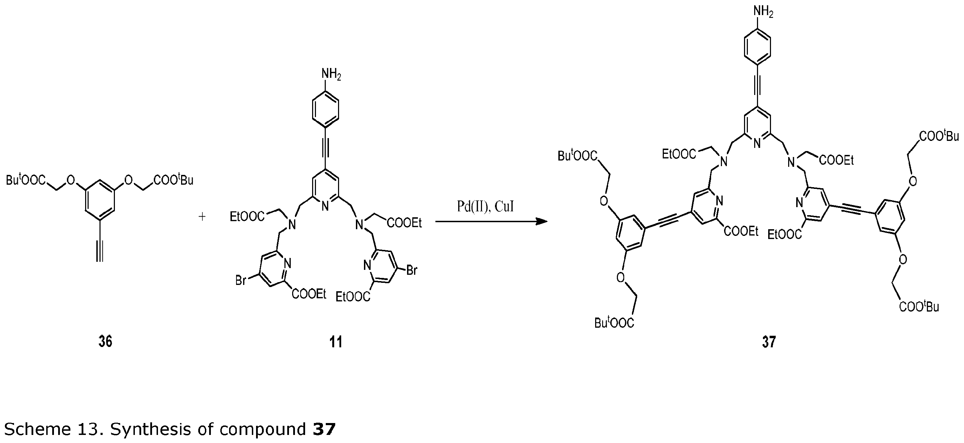

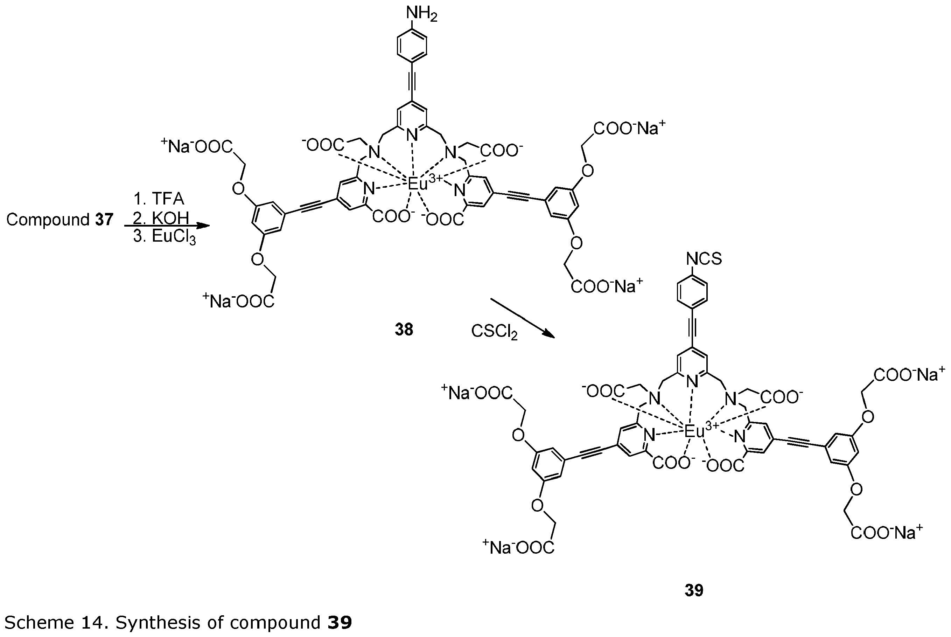

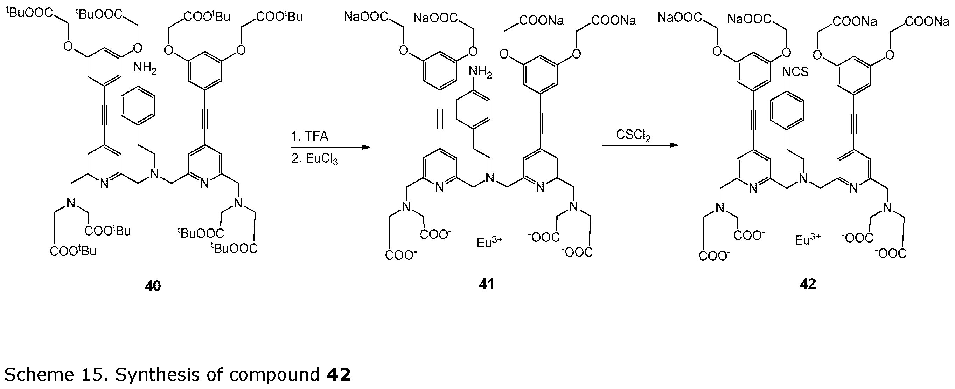

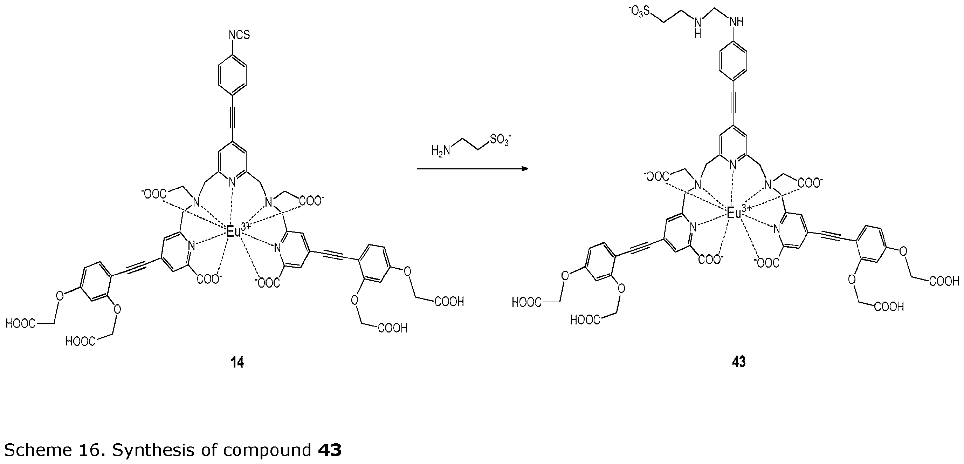

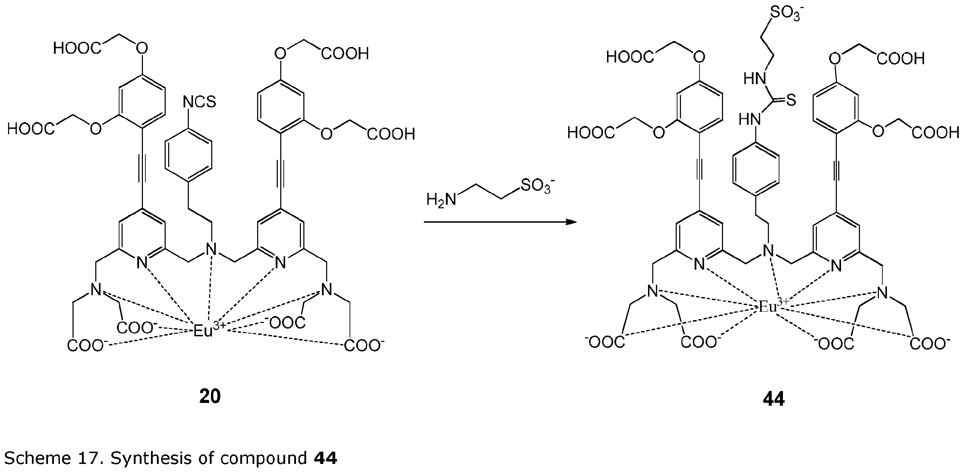

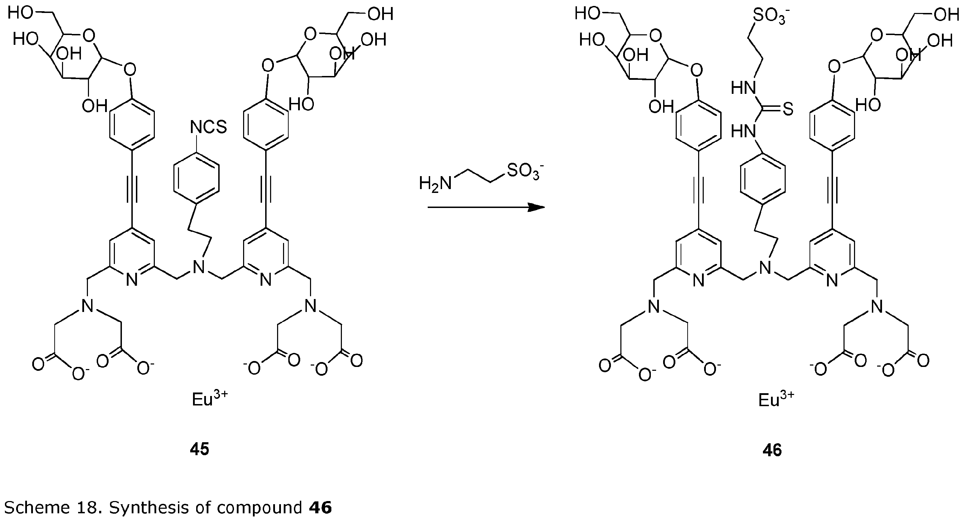

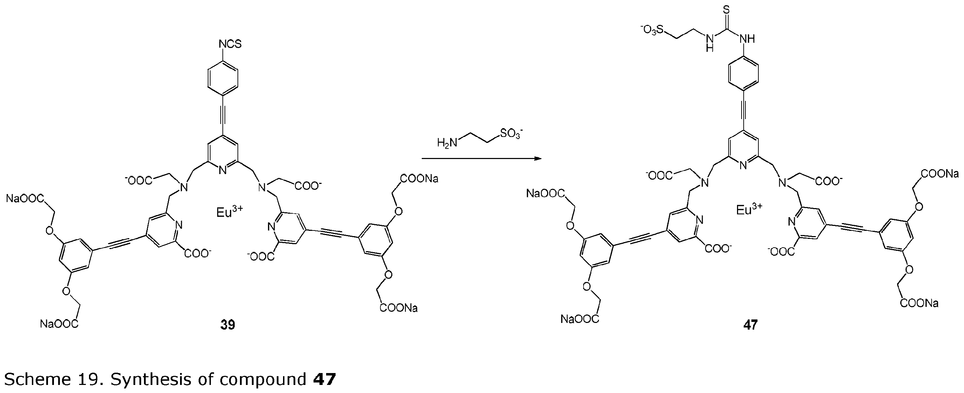

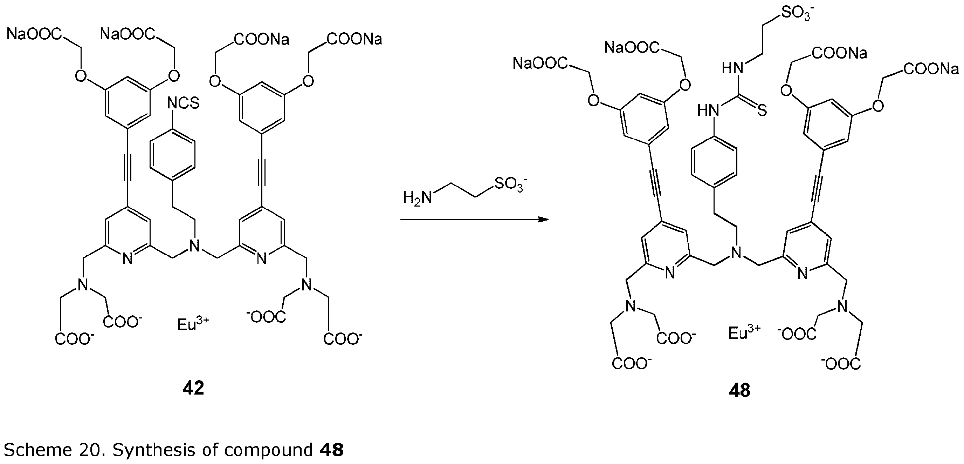

The excitation spectrum of the purified tracer antibody labelled with compound **14** was measured both in liquid phase and in dry state. For comparison also an excitation spectrum of the reference compound **45** is presented in Figure 2. (liquid phase measurement, dashed line).

The excitation spectrum of the antibody labelled with compound **14** shows clear red-shift compared to the reference compound in the excitation maximum.

### Example 44. Troponin I immunoassay

The performance of the new chelate (**14**) was evaluated also in sandwich immunoassay for cardiac troponin I. As a reference compound a TnI antibody labelled with compound **45** (labelling degree 10 Eu/IgG) was used. 10 µl of diluted tracer antibody (5ng/µl) and 20 µl of TnI standard solution were pipetted to a pre-coated assay well (single wells in 96 well plate format, wells coated with streptavidin and a biotinylated capture antibody against TnI, Innotrac Diagnostics). The reaction mixtures were incubated 20 min at 36°C with shaking. The wells were washed 6 times and dried prior to measurement with Victor™ Plate-fluorometer using a 340 nm excitation wavelength. The results are summarized in Table 5. Both A and B standards were measured in 12 replicates and other standards C-F in 6 replicates. The results are presented in Table 5.

**Table 5.**

| | **cTnl std** | **A** | **B** | **C** | **D** | **E** | **F** |
|---|---|---|---|---|---|---|---|
| | **(ng/ml)** | **0** | **0,022** | **0,08** | **0,75** | **5,34** | **41,6** |
| **Antibody labeled with compound 14** | **cps-blank** | 213 | 600 | 2108 | 17187 | 122616 | 866665 |
| **Reference antibody labeled with compound 45** | **cps-blank** | 183 | 267 | 906 | 7548 | 55086 | 427600 |
| **Antibody labeled with compound 14** | **cv%** | 6.6 | 5.9 | 5.5 | 2.1 | 2.3 | 0.7 |
| **Reference antibody labeled with compound 45** | **cv%** | 9.7 | 5.5 | 4.2 | 2.3 | 3.4 | 3.6 |

The results show that the chelate (14) provides the same signal levels with 1/10 amount of chelate/IgG when compared to the reference compound. In other words the compound according to present invention provides an order of magnitude more luminescence signal per chelate than the state of the art reference compound. Noteworthy is that the excitation wavelength was 340 nm which was standard setting in the plate-fluorometer. If the excitation would have taken place at 365 nm the difference between the two chelates can be assumed even larger in favor of the new chelate. Noteworthy is also that the obtained CV%'s and background signals were excellent for the new chelate.

### Example 45. Photo-physical properties of a novel chelate conjugated to taurine (chelate 31)

The prepared isothiocyante activated chelate (**30**) was conjugated to taurine as described above in Example 38. The product was purified with semi-preparative reversed phase HPLC (RP-18 column). Rf(HPLC)=14.3 min. UV/VIS=349 nm.After the product fractions were evaporated the residues were dissolved in 50 mM TRIS buffer.

The measured photo-physical properties excitation wavelengths (λ_{exc}), luminescence decay times (τ), molar absorptivities (ε), estimated luminescence yields (εΦ) of the novel chelate (**31**) in 50 mM TRIS buffer (pH 7.75) are in the Table 6.

**Table 6.**

| Chelate | ε | εϕ | Mean lifetime (ms) | Excitation max. (nm) |
|---|---|---|---|---|
| **31** | 64,000 | 16,400 | 0.70 | 343 |

### Example 46. Labelling of antibody with chelates 30

The TnI labeled antibodies were prepared as described in Example 42.

The measured photo-physical properties excitation wavelengths λ_{exc}), luminescence decay times (τ), molar absorptivities (ε), luminescence yields (εΦ) of the labelled cTnIs with the chelate **30** in 50 mM TRIS buffer (pH 7.75) in Table 7.

Dry measurements (**30** (dry)) represent estimated luminescence yields based on the signal measurements after dry immunoassay done as described in the Example 44.

**Table 7.**

| Labeled IgG with chelate | ε | εΦ | mean lifetime (ms) | excitation max. (nm) |
|---|---|---|---|---|
| **30** | 78,000 | 11,400 | 0,69 | 344 |
| dry | | 20,700 | | |

The luminescence yield of labeled IgG (30) in dry is surprisingly high, further the obtained excitation max is also high.

Overall, the results are promising and clearly demonstrate the high potential of this type of chelates.

## Claims

1. A luminescent lanthanide chelate comprising one or more chromophoric moieties of the formula (I) or of the formula (III): 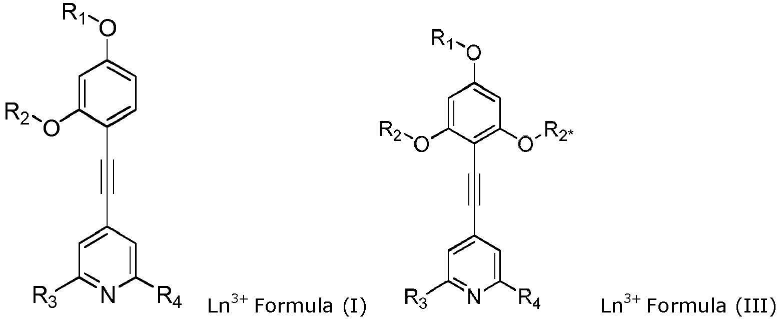 wherein R₁, R₂ and R₂*, when present, each independently are selected from -(CH₂)₁₋₆COOH, -(CH₂)₁₋₆COO⁻, -(CH₂)₁₋₆SO₃H, -(CH₂)₁₋₆SO₃⁻, -(CH₂CH₂O)₁₋₄OCH₂CH₂OH, -(CH₂CH₂O)₁₋₄OCH₂CH₂OCH₃, -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, -(CH₂)₁₋₆C(=O)R₅, and -(CH₂)₁₋₆-C₆H₄-R₅, wherein R₅ is selected from hydrogen, C₁₋₁₂-alkyl, -(CH₂)₁₋₆COOH, -(CH₂)₁₋₆COO⁻, -(CH₂)₁₋₆SO₃H, -(CH₂)₁₋₆SO₃⁻, a hydrophilic group (optionally including a spacer), a reactive group (optionally including a spacer), a polypeptide and a nucleotide, R₃ and R₄ each represent a bond between the chromophoric moiety and other moieties of the chelate, and Ln³⁺ is a lanthanide ion.

2. The luminescent lanthanide chelate according to claim 1, wherein R₁, R₂ and R_{2*}, when present, each independently are selected from -(CH₂)₁₋₆COOH, -(CH₂)₁₋₆COO⁻, -(CH₂)₁₋₆SO₃H, and -(CH₂)₁₋₆SO₃⁻.

3. The luminescent lanthanide chelate according to claim 2, wherein R₁, R₂ and R_{2*}, when present, each independently are selected from -CH₂-COOH and -CH₂-COO⁻.

4. The luminescent lanthanide chelate according to any one of the preceding claims, wherein the one or more chromophoric moieties are of the formula (I).

5. The luminescent lanthanide chelate according to any one of the preceding claims, wherein one or more chromophoric moieties are of the formula (III).

6. The luminescent lanthanide chelate according to any one of the preceding claims, wherein the lanthanide ion, Ln³⁺, is selected from europium(III), terbium(III), dysprosium(III) and samarium(III).

7. The luminescent lanthanide chelate according to claim 1, which has one of the structural formulas (A-I), (A-II), (B-I) or (B-II): 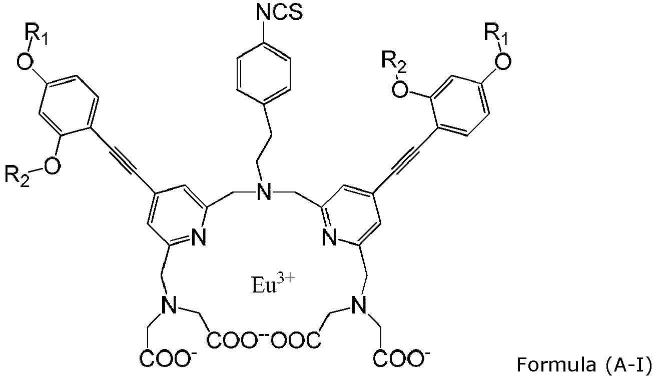 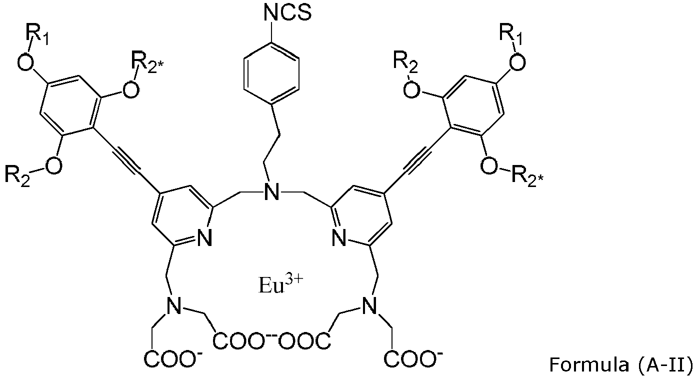 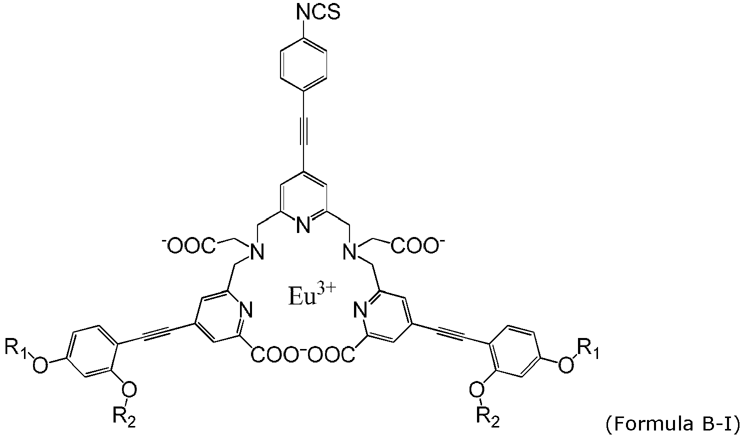 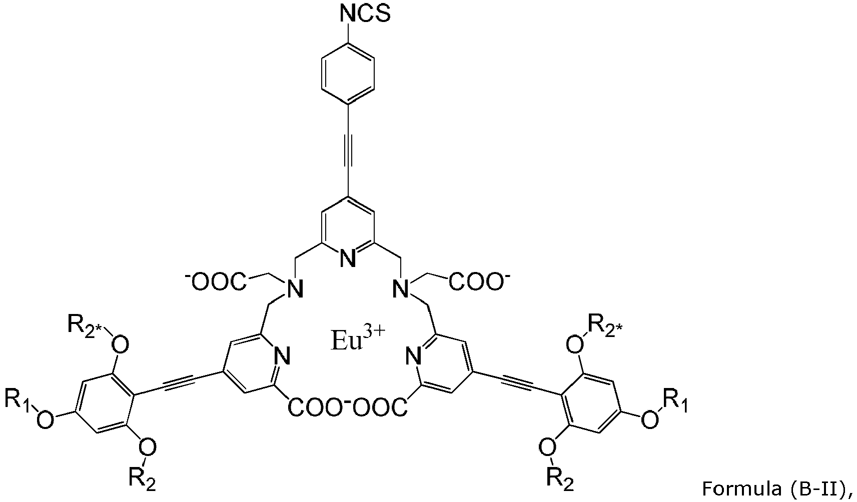 wherein the substituents R₁, R₂ and R_{2*}, when present, may each independently be selected from -(CH₂)₁₋₆COOH, -(CH₂)₁₋₆COO⁻, -(CH₂)₁₋₆SO₃H, -(CH₂)₁₋₆SO₃⁻, -(CH₂CH₂O)₁₋₄OCH₂CH₂OH, -(CH₂CH₂O)₁₋₄OCH₂CH₂OCH₃, -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, and -(CH₂)₁₋₆C(=O)R₅, wherein R₅ is selected from hydrogen, C₁₋₁₂-alkyl, -(CH₂)₁₋₆COOH, -(CH₂)₁₋₆COO⁻, -(CH₂)₁₋₆SO₃H, -(CH₂)₁₋₆SO₃⁻, a hydrophilic group (optionally including a spacer), a reactive group (optionally including a spacer), a polypeptide and a nucleotide.

8. A detectable molecule comprising a biospecific binding reactant conjugated to a luminescent lanthanide chelate of the formula (I) as defined in any one of claims 1-7.

9. The detectable molecule according to claim 8, wherein the biospecific binding reactant is selected from an antibody, an antigen, a receptor ligand, a specific binding protein, a DNA probe, a RNA probe, an oligopeptide, an oligonucleotide, a modified oligonucleotide, a modified polynucleotide, a protein, an oligosaccaride, a polysaccharide, a phospholipid, a PNA, a steroid, a hapten, a drug, a receptor binding ligand, and lectine.

10. The detectable molecule according to any of claims 8 and 9, wherein the biospecific binding reactant is an antibody.

11. A lanthanide chelating ligand comprising one or more chromophoric moieties of the formula (II) or of the formula (IV) 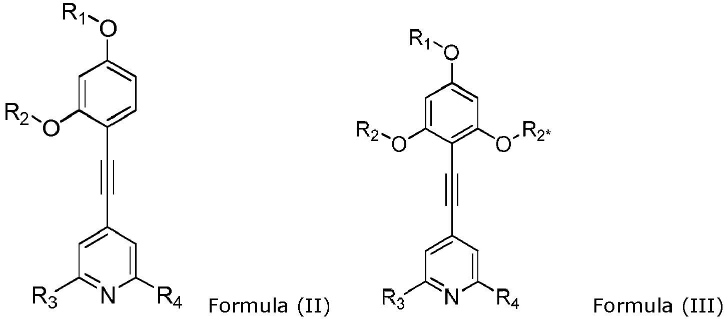 wherein each of R₁, R₂, R_{2*}, R₃, and R₄ represents the groups R₁, R₂, R_{2*}, R₃, and R₄, respectively, as defined in any one of claims 1-7.

12. A method of carrying out a biospecific binding assay, said method comprising the steps of:
a) forming a biocomplex between an analyte and a biospecific binding reactant labelled with a luminescent lanthanide chelate according to any one of claims 1-7;
b) exciting said biocomplex with radiation having an excitation wavelength, thereby forming an excited biocomplex; and
c) detecting emission radiation emitted from said excited biocomplex.

13. Use of a detectable molecule according to any one of claims 8-10 in a specific bioaffinity based binding assay utilizing time-resolved fluorometric determination of a specific luminescence.

14. A solid support material conjugated with a luminescent lanthanide chelate according to any of the claims 1-7 or a lanthanide chelating ligand according to claim 11.

## Patentansprüche

1. Lumineszierendes Lanthanidchelat, umfassend eine oder mehrere chromophore Einheiten der Formel (I) oder der Formel (III): 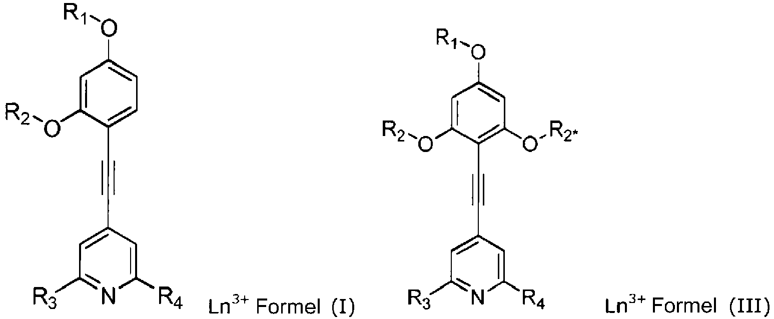 worin R₁, R₂ und R_{2*}, wenn vorhanden, jeweils unabhängig ausgewählt sind aus -(CH₂)₁₋₆COOH, -(CH₂)₁₋₆COO⁻, -(CH₂)₁₋₆SO₃H, -(CH₂)₁₋₆SO₃⁻,-(CH₂CH₂O)₁₋₄OCH₂CH₂OH, -(CH₂CH₂O)₁₋₄OCH₂CH₂OCH₃, -(CH₂)₁-₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, -(CH₂)₁₋₆C(=O)R₅, und -(CH₂)₁₋₆-C₆H₄-R₅, worin R₅ ausgewählt ist aus Wasserstoff, C₁₋₁₂-Alkyl, -(CH₂)₁₋₆COOH, -(CH₂)₁₋₆COO⁻, -(CH₂)₁₋₆SO₃H, -(CH₂)₁₋₆SO₃⁻, einer hydrophilen Gruppe (die optional einen Spacer beinhaltet), einer reaktiven Gruppe (die optional einen Spacer beinhaltet), einem Polypeptid und einem Nukleotid, R₃ und R₄ jeweils für eine Bindung zwischen der chromophoren Einheit und anderen Einheiten des Chelats stehen, und Ln³⁺ ein Lanthanidion ist.

2. Lumineszierendes Lanthanidchelat nach Anspruch 1, wobei R₁, R₂ und R_{2*}, wenn vorhanden, jeweils unabhängig ausgewählt sind aus -(CH₂)₁₋₆COOH,-(CH₂)₁₋₆COO⁻, -(CH₂)₁₋₆SO₃H, und -(CH₂)₁₋₆SO₃⁻.

3. Lumineszierendes Lanthanidchelat nach Anspruch 2, wobei R₁, R₂ und R_{2*}, wenn vorhanden, jeweils unabhängig ausgewählt sind aus -CH₂-COOH und-CH₂-COO⁻.

4. Lumineszierendes Lanthanidchelat nach einem der vorstehenden Ansprüche, wobei eine oder mehrere chromophore Einheiten von der Formel (I) sind.

5. Lumineszierendes Lanthanidchelat nach einem der vorstehenden Ansprüche, wobei eine oder mehrere der chromophoren Einheiten von der Formel (III) sind.

6. Lumineszierendes Lanthanidchelat nach einem der vorstehenden Ansprüche, wobei das Lanthanidion, Ln³⁺, ausgewählt ist aus Europium(III), Terbium(III), Dysprosium(III) und Samarium(III).

7. Lumineszierendes Lanthanidchelat nach Anspruch 1, das eine der folgenden strukturellen Formeln (A-I), (A-II), (B-I) oder (B-II) aufweist: 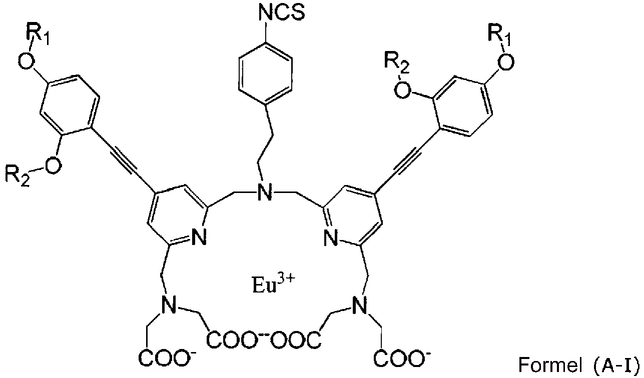 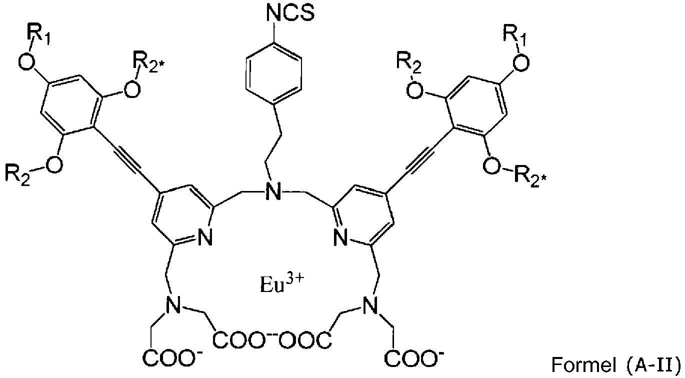 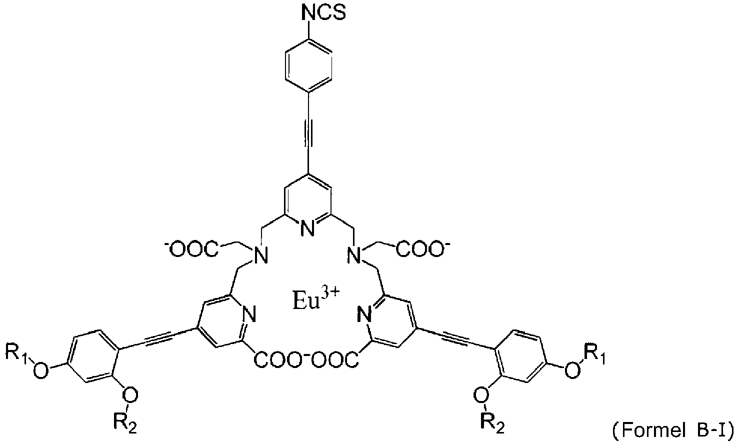 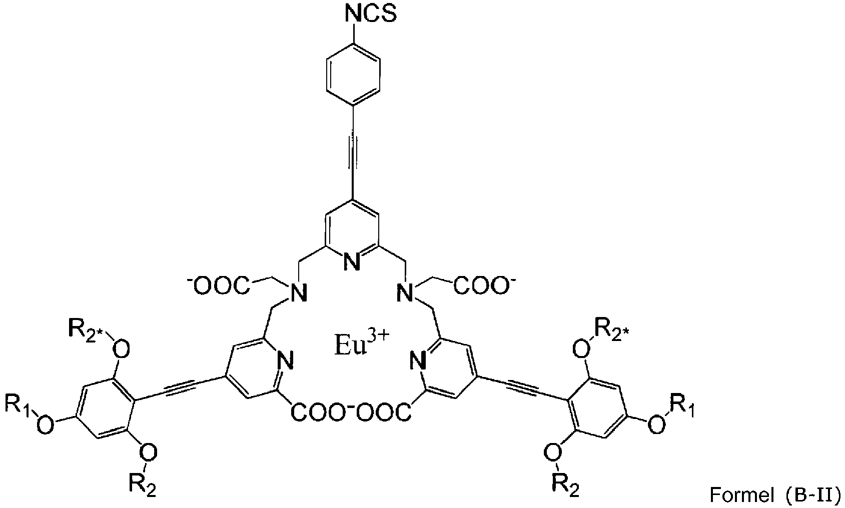 wobei die Substituenten R₁, R₂ und R_{2*}, wenn vorhanden, jeweils unabhängig ausgewählt werden können aus -(CH₂)₁₋₆COOH, -(CH₂)₁₋₆COO⁻, -(CH₂)₁₋₆SO₃H,-(CH₂)₁₋₆SO₃⁻, -(CH₂CH₂O)₁₋₄OCH₂CH₂OH, -(CH₂CH₂O)₁₋₄OCH₂CH₂OCH₃,-(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, und -(CH₂)₁₋₆C(=O)R₅, worin R₅ ausgewählt ist aus Wasserstoff, C₁₋₁₂-Alkyl, -(CH₂)₁₋₆COOH, -(CH₂)₁₋₆COO⁻, -(CH₂)₁₋₆SO₃H, -(CH₂)₁₋₆SO₃⁻, einer hydrophilen Gruppe (die optional einen Spacer beinhaltet), einer reaktiven Gruppe (die optional einen Spacer beinhaltet), einem Polypeptid und einem Nukleotid.

8. Detektierbares Molekül, umfassend ein biospezifisches Bindungsreaktant, das an ein lumineszierendes Lanthanidchelat der Formel (I) wie in einem der Ansprüche 1 bis 7 definiert, bindet.

9. Detektierbares Molekül nach Anspruch 8, wobei das biospezifische Bindungsreaktant ausgewählt ist aus einem Antikörper, einem Antigen, einem Rezeptorliganden, einem spezifischen Bindungsprotein, einer DNA-Sonde, einer RNA-Sonde, einem Oligopeptid, einem Oligonukleotid, einem modifizierten Oligonukleotid, einem modifizierten Polynukleotid, einem Protein, einem Oligosaccharid, einem Polysaccharid, einem Phospholipid, einem PNA, einem Steroid, einem Hapten, einem Arzneimittel, einem Rezeptorbindungsliganden und Lektin.

10. Detektierbares Molekül nach einem der Ansprüche 8 und 9, wobei der spezifische Bindungsreaktant ein Antikörper ist.

11. Lanthanidchelatligand, umfassend eine oder mehrere chromophore Einheiten der Formel (II) oder der Formel (IV) 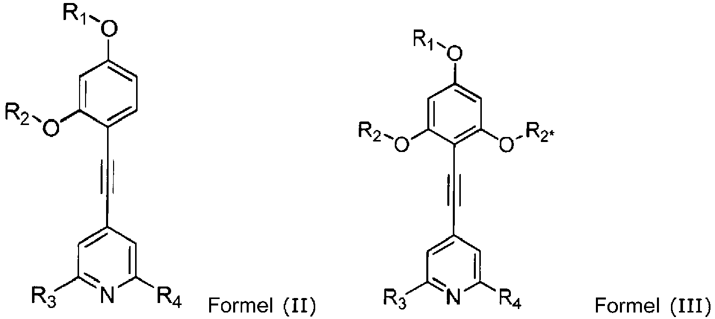 wobei jedes von R₁, R₂, R_{2*}, R₃, und R₄ für die Gruppen R₁, R₂, R_{2*}, R₃, bzw. R₄, wie in einem der Ansprüche 1 bis 7 definiert, steht.

12. Verfahren zur Durchführung eines biospezifischen Bindungsassays, wobei das Verfahren die folgenden Schritte umfasst:
a) Bilden eines Biokomplexes zwischen einem Analyten und einem biospezifischen Bindungsreaktanten, der mit einem lumineszierenden Lanthanidchaleat nach einem der Ansprüche 1 bis 7 markiert ist;
b) Anregen des Biokomplexes mit Strahlung mit einer Anregungswellenlänge, wodurch ein angeregter Biokomplex gebildet wird; und
c) Detektieren der Emissionsstrahlung, die von dem angeregten Biokomplex emittiert wird.

13. Verwendung eines detektierbaren Moleküls nach einem der Ansprüche 8 bis 10 in einem Bindungsassay, der auf einer spezifischen Bioaffinität basiert, unter Verwendung von zeitaufgelöster, fluorometrischer Bestimmung einer spezifischen Lumineszenz.

14. Festes Trägermaterial, das mit einem lumineszierenden Lanthanidchelat nach einem der Ansprüche 1 bis 7 oder einem Lanthanidchelat nach Anspruch 11 konjugiert ist.

## Revendications

1. Chélate de lanthanide luminescent comprenant un ou plusieurs fragments chromophores de formule (I) ou de formule (III) : 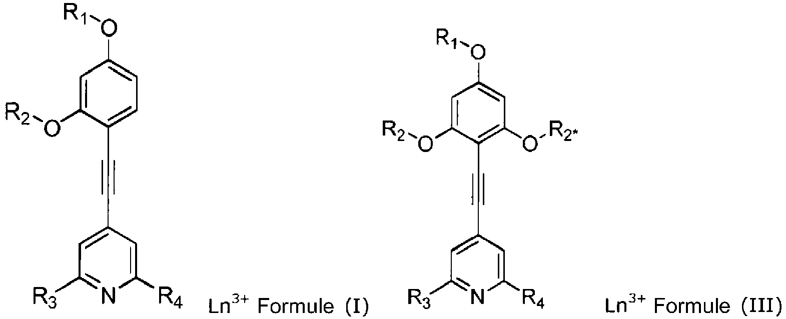 dans lesquelles R₁, R₂ et R_{2*}, lorsqu'ils sont présents, sont choisis chacun indépendamment parmi -(CH₂)₁₋₆COOH, -(CH₂)₁₋₆COO⁻, -(CH₂)₁₋₆SO₃H, -(CH₂)₁₋₆SO₃⁻, -(CH₂CH₂O)₁₋₄OCH₂CH₂OH, -(CH₂CH₂O)₁₋₄OCH₂CH₂OCH₃, -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅, -(CH₂)₁₋₆C(=O)R₅ et -(CH₂)₁₋₆-C₆H₄-R₅, où R₅ est choisi parmi l'hydrogène, un alkyle en C₁₋₁₂, -(CH₂)₁₋₆COOH, -(CH₂)₁₋₆COO⁻, -(CH₂)₁₋₆SO₃H, -(CH₂)₁₋₆SO₃⁻, un groupe hydrophile (comprenant éventuellement un espaceur), un groupe réactif (comprenant éventuellement un espaceur), un polypeptide et un nucléotide, R₃ et R₄ représentent chacun une liaison entre le fragment chromophore et d'autres fragments du chélate et Ln³⁺ est un ion lanthanide.

2. Chélate de lanthanide luminescent selon la revendication 1, dans lequel R₁, R₂ et R_{2*}, lorsqu'ils ils sont présents, sont choisis chacun indépendamment parmi - (CH₂)₁₋₆COOH, -(CH₂)₁₋₆COO⁻, -(CH₂)₁₋₆SO₃H et -(CH₂)₁₋₆SO₃⁻.

3. Chélate de lanthanide luminescent selon la revendication 2, dans lequel R₁, R₂ et R_{2*}, lorsqu'ils sont présents, sont choisis chacun indépendamment parmi -CH₂-COOH et -CH₂-COO⁻.

4. Chélate de lanthanide luminescent selon l'une quelconque des revendications précédentes, dans lequel le ou les fragment(s) chromophores sont de formule (I).

5. Chélate de lanthanide luminescent selon l'une quelconque des revendications précédentes, dans lequel le ou les fragment(s) chromophores sont de formule (III).

6. Chélate de lanthanide luminescent selon l'une quelconque des revendications précédentes, dans lequel l'ion lanthanide, Ln³⁺, est choisi parmi l'europium(III), le terbium(III), le dysprosium(III) et le samarium(III).

7. Chélate de lanthanide luminescent selon la revendication 1, qui présente l'une des formules structurales (A-I), (A-II), (B-I) ou (B-II) : 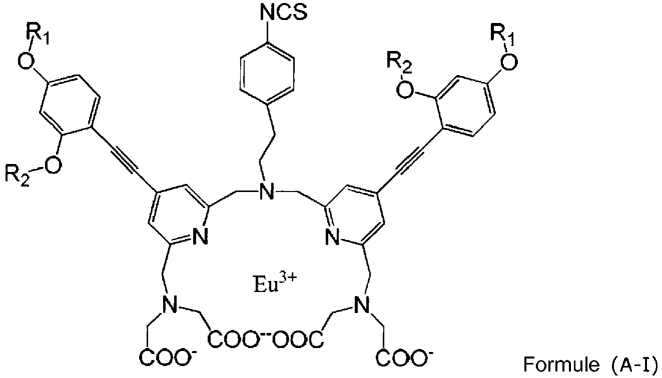 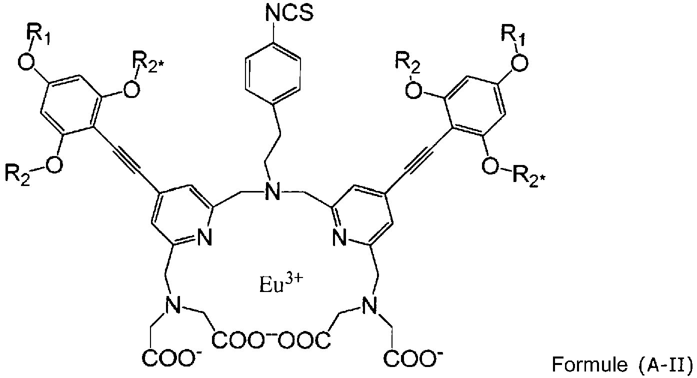 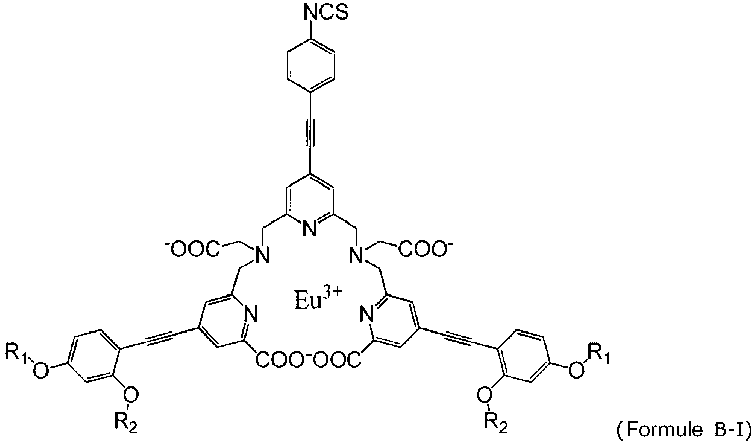 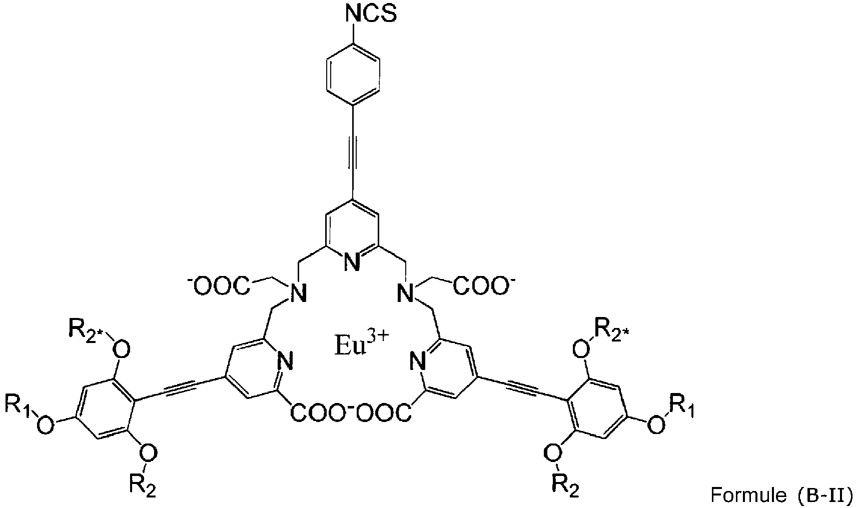 dans lesquelles les substituants R₁, R₂ et R_{2*}, lorsqu'ils sont présents, peuvent être choisis chacun indépendamment parmi -(CH₂)₁₋₆COOH, -(CH₂)₁₋₆COO⁻, -(CH₂)₁₋₆SO₃H, -(CH₂)₁₋₆SO₃⁻, -(CH₂CH₂O)₁₋₄OCH₂CH₂OH, -(CH₂CH₂O)₁₋₄OCH₂CH₂OCH₃, -(CH₂)₁₋₆NHC(=O)R₅, -(CH₂)₁₋₆NCH₃C(=O)R₅, -(CH₂)₁₋₆C(=O)NHR₅, -(CH₂)₁₋₆C(=O)NCH₃R₅, -(CH₂)₁₋₆NHC(=O)NHR₅, -(CH₂)₁₋₆NHC(=S)NHR₅ et -(CH₂)₁₋₆C(=O)R₅, où R₅ est choisi parmi l'hydrogène, un alkyle en C₁₋₁₂, -(CH₂)₁₋₆COOH, -(CH₂)₁₋₆COO⁻, -(CH₂)₁₋₆SO₃H, -(CH₂)₁₋₆SO₃⁻, un groupe hydrophile (comprenant éventuellement un espaceur), un groupe réactif (comprenant éventuellement un espaceur), un polypeptide et un nucléotide.

8. Molécule détectable comprenant un réactif de liaison biospécifique conjugué à un chélate de lanthanide luminescent de formule (I) ainsi que défini dans l'une quelconque des revendications 1 à 7.

9. Molécule détectable selon la revendication 8, dans laquelle le réactif de liaison biospécifique est choisi parmi un anticorps, un antigène, un ligand de récepteur, une protéine de liaison spécifique, une sonde à ADN, une sonde à ARN, un oligopeptide, un oligonucléotide, un oligonucléotide modifié, un polynucléotide modifié, une protéine, un oligosaccharide, un polysaccharide, un phospholipide, un ANP, un stéroïde, un haptène, un médicament, un ligand de liaison à un récepteur et une lectine.

10. Molécule détectable selon l'une quelconque des revendications 8 et 9, dans laquelle le réactif de liaison biospécifique est un anticorps.

11. Ligand chélatant les lanthanides comprenant un ou plusieurs fragments chromophores de formule (II) ou de formule (IV) 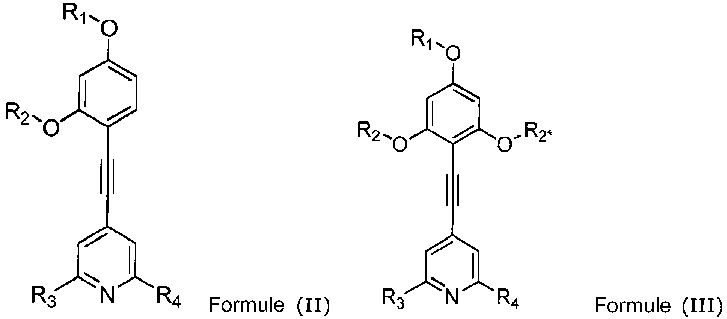 dans lequel chacun parmi R₁, R₂, R_{2*}, R₃ et R₄ représente respectivement les groupes R₁, R₂, R_{2*}, R₃ et R₄ ainsi que définis dans l'une quelconque des revendications 1 à 7.

12. Procédé de réalisation d'un essai de liaison biospécifique, ledit procédé comprenant les étapes consistant à :
a) former un biocomplexe entre un analyte et un réactif de liaison biospécifique marqué au moyen d'un chélate de lanthanide luminescent selon l'une quelconque des revendications 1 à 7 ;
b) exciter ledit biocomplexe avec un rayonnement ayant une longueur d'onde d'excitation, formant ainsi un biocomplexe excité ; et
c) détecter un rayonnement d'émission émis par ledit biocomplexe excité.

13. Utilisation d'une molécule détectable selon l'une quelconque des revendications 8 à 10 dans un essai de liaison basé sur une bioaffinité spécifique utilisant une détermination fluorométrique à résolution temporelle d'une luminescence spécifique.

14. Matériau support solide conjugué avec un chélate de lanthanide luminescent selon l'une quelconque des revendications 1 à 7 ou un ligand chélatant les lanthanides selon la revendication 11.
